# EUROPEAN PATENT APPLICATION

(11) **EP 4 696 306 A1**
(43) Date of publication of application: **18.02.2026**
(21) Application number: 24797113.8
(22) Date of filing: 25.04.2024
(51) Int. Cl.: A61K 31/155, A61K 31/53, A61K 31/661, A61P 1/16, A61P 17/06, A61P 43/00

(54) **ADENOSINE TRIPHOSPHATE SECRETION INHIBITOR, HEPATIC STELLATE CELL ACTIVATION INHIBITOR, HEPATIC STELLATE CELL DEACTIVATOR, HEPATIC FIBROSIS THERAPEUTIC AGENT, NON-ALCOHOLIC STEATOHEPATITIS THERAPEUTIC AGENT, PSORIASIS THERAPEUTIC AGENT, AND USE OF COMPOUND FOR PRODUCING ADENOSINE TRIPHOSPHATE SECRETION INHIBITOR, HEPATIC STELLATE CELL ACTIVATION INHIBITOR, HEPATIC STELLATE CELL DEACTIVATOR, HEPATIC FIBROSIS THERAPEUTIC AGENT, NON-ALCOHOLIC STEATOHEPATITIS THERAPEUTIC AGENT, AND PSORIASIS THERAPEUTIC AGENT**

(30) Priority: 28.04.2023 JP 2023074235
(71) Applicant: KURUME UNIVERSITY, Kurume-shi Fukuoka 830-0011 (JP)
(72) Inventor: NOMURA Masatoshi, Kurume-shi, Fukuoka 830-0011 (JP); MORIYAMA Yoshinori, Kurume-shi, Fukuoka 830-0011 (JP); HASUZAWA Nao, Kurume-shi, Fukuoka 830-0011 (JP); MORIYAMA Sawako, Kurume-shi, Fukuoka 830-0011 (JP)
(74) Representative: v. Bezold & Partner Patentanwälte - PartG mbB
(86) International application number: PCT/JP2024/016206
(87) International publication number: WO 2024/225368

(57) **Abstract**

An adenosine triphosphate secretion inhibitor includes: imeglimin, metformin, phosphoenolpyruvic acid, an analog of phosphoenolpyruvic acid, or a pharmaceutically acceptable salt thereof.

## Description

### Technical Field

The present disclosure relates to an adenosine triphosphate secretion inhibitor, a hepatic stellate cell activation inhibitor, a hepatic stellate cell deactivator, a hepatic fibrosis therapeutic agent, a non-alcoholic steatohepatitis therapeutic agent, and a psoriasis therapeutic agent, as well as to the use of a compound for the production of an adenosine triphosphate secretion inhibitor, a hepatic stellate cell activation inhibitor, a hepatic stellate cell deactivator, a hepatic fibrosis therapeutic agent, a non-alcoholic steatohepatitis therapeutic agent, and a psoriasis therapeutic agent.

### Background Art

Purinergic chemical transmission is a mode of intercellular communication in which adenosine triphosphate (ATP) loaded in secretory vesicles is secreted in response to various stimuli and then binds to purinergic receptors, resulting in signal transduction. Although ATP is well known for its role as an energy currency in the cell, it functions as a signal transducer in purinergic chemical transmission.

Secretion of ATP is initiated by loading of the ATP into secretory vesicles, followed by fusion of the secretory vesicles with the cell membrane, resulting in extracellular release of the ATP (exocytosis). Loading of ATP into the vesicles involves vesicular nucleotide transporter (VNUT) and vacuolar ATPase (V-ATPase). V-ATPase transports H⁺ into vesicles to accumulate H⁺ motive force in the vesicles. Subsequently, VNUT transports ATP into the vesicles using the H⁺ motive force. According to Non Patent Literature 1, secretory vesicles loaded with ATP fuse with the cell membrane through the action of a group of soluble NSF attachment protein receptor (SNARE) proteins, resulting in exocytosis of ATP.

In recent years, purinergic chemical transmission has been found to be involved in a variety of diseases and symptoms, including inflammatory and neuropathic pains, ulcerative colitis, non-alcoholic hepatitis, fibrosis of hepatocytes, type 2 diabetes, chronic inflammation, blood coagulation disorder, lower urinary tract dysfunction, and pruritus caused by chemical substances or bacterial infection. Inhibition of ATP secretion in purinergic chemical transmission has been reported to markedly reduce these symptoms. Furthermore, studies on purinergic receptors have suggested that such inhibition is potentially applicable in Parkinson's disease, Alzheimer's disease, epilepsy, atopic dermatitis, hepatic fibrosis, psoriasis, atherosclerosis, and cancer.

Specifically, purinergic chemical transmission also plays an important role in the fibrosis of various organs including the liver. Extracellular ATP and its metabolites act as signaling molecules via purinergic receptors to influence the processes of tissue repair, healing, and scarring. In particular, adenosine generated from extracellular ATP by ectonucleotidase, CD39, and CD73 promotes fibrosis in the skin, heart, liver, and lungs.

Fibrosis is characterized by excessive accumulation of collagen and other components in the extracellular matrix (ECM) during wound healing and tissue processes. In hepatic fibrosis, ECM-producing myofibroblasts are primarily derived from hepatic stellate cells (HSCs) located in the perisinusoidal space of the liver. Specifically, when HSCs are stimulated by various molecules released from hepatocytes and immune cells as a result of certain liver injury, the HSCs transform into proliferative, ECM-producing myofibroblasts, which actively synthesize and secrete ECM, fibrogenic factors, chemokines, and cytokines. These cause further liver damage. As described in Non Patent Literature 2, activation of HSCs is a key event leading to hepatic fibrosis. Therefore, inhibition of HSC activation and induction of HSC deactivation are considered to have a direct effect on the prevention of fibrosis.

Non Patent Literature 3 demonstrates that VNUT-deficient mice show significant improvement in inflammatory cell infiltration and fibrosis in a high-fat diet-induced non-alcoholic steatohepatitis (NASH) model. Furthermore, Non Patent Literature 4 discloses that administration of clodronate, a VNUT inhibitor, improves the NASH activity score, inflammatory cell infiltration, and hepatic fibrosis in a methionine- and choline-deficient (MCD) diet-induced NASH model.

Further, part of ATP secreted from cells is metabolized extracellularly to ADP and adenosine. Extracellular ATP and its metabolites function as intercellular signaling substances. They transmit nociceptive stimuli to target cells via purinergic receptors, and enhance the secretion of inflammatory cytokines, resulting in induction of an inflammatory response. Since increased ATP secretion leads to increased cytokine secretion, ATP secretion is responsible for triggering inflammatory reactions.

Thus, ATP secretion inhibitors with fewer side effects are useful for the treatment or amelioration of various diseases and symptoms. Patent Literature 1 describes a pharmaceutical composition including, for example, clodronate, which is a compound having VNUT inhibitory activity.

### Citation List

### Patent Literature

Patent Literature 1: Unexamined Japanese Patent Application Publication No. 2019-014684

### Non Patent Literature

Non Patent Literature 1: Nao Hasuzawa et al., 2020, Physiopathological roles of vesicular nucleotide transporter (VNUT), an essential component for vesicular ATP release, Biochim Biophys Acta Biomembranes, 1862(12), 183408
Non Patent Literature 2: Juliane S Troeger et al., 2012, Deactivation of hepatic stellate cells during liver fibrosis resolution in mice, Gastroenterology, 143 (4), 1073-1083
Non Patent Literature 3: Keita Tatsushima et al., 2021, Vesicular ATP release from hepatocytes plays a role in the progression of nonalcoholic steatohepatitis, Biochem Biophys Acta Mol Basis of Dis. 1867 (3), 166013.
Non Patent Literature 4: Nao Hasuzawa et al., 2021, Clodronate, an inhibitor of the vesicular nucleotide transporter, ameliorates steatohepatitis and acute liver injury, Sci rep, 11 (1), 5192

### Summary of Invention

### Technical Problem

However, clodronate is known to show extremely low absorption, and is also known to accumulate in bone, which leads to jaw osteonecrosis, a serious side effect. Therefore, in the pharmaceutical industry, there is a demand for a compound that has an ATP secretion inhibitory effect and that does not cause side effects. In addition, although clodronate has long been used as a drug for osteoporosis in the United Kingdom and Canada, it has not been used in Japan, the United States, or China, and thus requires new approval as a pharmaceutical in these countries, which is disadvantageous.

The present disclosure was made in view of the above circumstances, and aims to provide an ATP secretion inhibitor, a hepatic stellate cell activation inhibitor, a hepatic stellate cell deactivator, a hepatic fibrosis therapeutic agent, a non-alcoholic steatohepatitis therapeutic agent, and a psoriasis therapeutic agent each of which is capable of inhibiting ATP secretion in purinergic chemical transmission and has minimal side effects.

### Solution to Problem

The present inventors identified a compound having an ATP secretion inhibitory effect in purinergic chemical transmission, thereby accomplishing the present disclosure. Furthermore, the present inventors discovered that the compound inhibits HSC activation or induces HSC deactivation and that the compound is effective for treatment of hepatic fibrosis and NASH. In addition, the present inventors discovered that the compound is effective for treatment of, and suppression of relapse of, psoriasis.

An adenosine triphosphate secretion inhibitor according to a first aspect of the present disclosure includes:
imeglimin, metformin, phosphoenolpyruvic acid, an analog of phosphoenolpyruvic acid, or a pharmaceutically acceptable salt thereof.

In this case, the adenosine triphosphate secretion inhibitor according to the first aspect may be used for prevention, amelioration, or treatment of a disease or symptom related to purinergic chemical transmission.

Further, the analog may be
L-2-phosphoglyceric acid, D-2-phosphoglyceric acid, or 3-phosphoglyceric acid.

A hepatic stellate cell activation inhibitor according to a second aspect of the present disclosure includes:
imeglimin, metformin, phosphoenolpyruvic acid, an analog of phosphoenolpyruvic acid, or a pharmaceutically acceptable salt thereof.

A hepatic stellate cell deactivator according to a third aspect of the present disclosure includes:
imeglimin, metformin, phosphoenolpyruvic acid, an analog of phosphoenolpyruvic acid, or a pharmaceutically acceptable salt thereof.

A hepatic fibrosis therapeutic agent according to a fourth aspect of the present disclosure includes:
imeglimin, metformin, phosphoenolpyruvic acid, an analog of phosphoenolpyruvic acid, or a pharmaceutically acceptable salt thereof.

A non-alcoholic steatohepatitis therapeutic agent according to a fifth aspect of the present disclosure includes:
imeglimin, metformin, phosphoenolpyruvic acid, an analog of phosphoenolpyruvic acid, or a pharmaceutically acceptable salt thereof.

A psoriasis therapeutic agent according to a sixth aspect of the present disclosure includes:
imeglimin, metformin, phosphoenolpyruvic acid, an analog of phosphoenolpyruvic acid, or a pharmaceutically acceptable salt thereof.

Use according to a seventh aspect of the present disclosure is
use of imeglimin, metformin, phosphoenolpyruvic acid, an analog of phosphoenolpyruvic acid, or a pharmaceutically acceptable salt thereof for the production of an adenosine triphosphate secretion inhibitor, a hepatic stellate cell activation inhibitor, a hepatic stellate cell deactivator, a hepatic fibrosis therapeutic agent, a non-alcoholic steatohepatitis therapeutic agent, or a psoriasis therapeutic agent.

### Advantageous Effects of Invention

The present disclosure provides an ATP secretion inhibitor, a hepatic stellate cell activation inhibitor, a hepatic stellate cell deactivator, a hepatic fibrosis therapeutic agent, a non-alcoholic steatohepatitis therapeutic agent, and a psoriasis therapeutic agent each of which is capable of inhibiting ATP secretion in purinergic chemical transmission and has minimal side effects.

### Brief Description of Drawings

FIG. 1 is a diagram illustrating the ATP secretion in Example 1;
FIG. 2 is a diagram illustrating the ATP secretion in Example 2;
FIG. 3 is a diagram illustrating the ATP secretion in Example 3;
FIG. 4 is a diagram illustrating the ATP secretion in Example 4;
FIG. 5 is a diagram illustrating the ATP uptake in Example 5;
FIG. 6 is a diagram illustrating the ATP uptake in Example 6;
FIG. 7 is a diagram illustrating the ATP secretion in Example 7;
FIG. 8 is a diagram illustrating the ATP secretion in Example 8;
FIG. 9 is a diagram illustrating the quantified amount of ATP in Example 9;
FIG. 10 is a diagram illustrating the ATP uptake in Example 10;
FIG. 11 is a diagram illustrating the dopamine uptake in Example 10;
FIG. 12 is a diagram illustrating the relative proton transport activity in Example 10;
FIG. 13 is a diagram illustrating a microscopic image of human epidermal keratinocytes in Example 11;
FIG. 14 is a diagram illustrating a microscopic image of human epidermal keratinocytes in Example 12;
FIG. 15 is a diagram illustrating comparison of the expression level of the *VNUT* gene in the liver based on microarray data in Example 13;
FIG. 16 is a diagram illustrating the results of immunostaining in Example 13;
FIG. 17 is a diagram illustrating immunofluorescence microscopic images in Example 13;
FIG. 18 is a diagram illustrating the Pearson's correlation coefficient between localization of VNUT and reactivity with each protein in Example 13;
FIG. 19 is a diagram illustrating images obtained by correlative light and electron microscopy (CLEM) in Example 13;
FIG. 20 is a diagram illustrating changes in the ATP secretion from LX-2 cells over time following the addition of ionomycin in Example 14;
FIG. 21 is a diagram illustrating the temperature dependence and the Ca²⁺ dependence of ATP secretion in Example 14;
FIG. 22 is a diagram illustrating the expression level of the *VNUT* gene as observed when RNAi was used in Example 14;
FIG. 23 is a diagram illustrating inhibition of ATP secretion by inhibition of expression of the *VNUT* gene in Example 14;
FIG. 24 is a diagram illustrating inhibition of ATP secretion by clodronate in Example 14;
FIG. 25 is a diagram illustrating induction of ATP secretion by serotonin and inhibition of ATP secretion by clodronate in Example 14;
FIG. 26 is a diagram illustrating inhibition of ATP secretion by imeglimin in Example 14;
FIG. 27 is a diagram illustrating the results of hematoxylin-eosin (HE) staining, picrosirius red (PSR) staining, and immunostaining in Example 15;
FIG. 28 is a diagram illustrating the fibrosis score in Example 15;
FIG. 29 is a diagram illustrating the results of immunostaining for α-SMA in liver sections in Example 16;
FIG. 30 is a diagram illustrating immunostaining and fluorescence microscopic images for α-SMA in liver sections in Example 16;
FIG. 31 is a diagram illustrating the results of Western blotting in Example 16;
FIG. 32 is a diagram illustrating ATP secretion in Example 17;
FIG. 33 is a diagram illustrating the morphology of mouse auricles in Example 18;
FIG. 34 is a diagram illustrating changes in the thickness of mouse auricles over time in Example 18;
FIG. 35 is a diagram illustrating tissues stained with HE in Example 18;
FIG. 36 is a diagram illustrating localization of transglutaminase 1 (TMG1)-positive cells in sections of mouse auricles in Example 18;
FIG. 37 is a diagram illustrating changes in the thickness of mouse auricles over time in Example 19;
FIG. 38 is a diagram illustrating changes in the erythema score over time in Example 19;
FIG. 39 is a diagram illustrating changes in the desquamation score over time in Example 19;
FIG. 40 is a diagram illustrating changes in the infiltration score over time in Example 19;
FIG. 41 is a diagram illustrating changes in the psoriasis area and severity index (PASI) score in Example 19;
FIG. 42 is a diagram illustrating localization of transglutaminase 1 (TMG1)-positive cells in sections of mouse auricles on Day 8 in Example 19;
FIG. 43 is a diagram illustrating changes in the thickness of mouse auricles over time in Example 20; and
FIG. 44 is a diagram illustrating changes in the thickness of mouse auricles over time in Example 21.

### Description of Embodiments

The following is a detailed description of embodiments of the present disclosure. The present disclosure is not limited by the following embodiments and drawings. In the following embodiments, expressions such as "have", "include", or "contain" also encompass the meaning of "consist of' or "be constituted by".

The ATP secretion inhibitor according to the present embodiment includes imeglimin, metformin, phosphoenolpyruvic acid (PEP), a PEP analog, or a pharmaceutically acceptable salt thereof.

The structure of imeglimin is shown below.

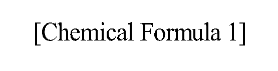

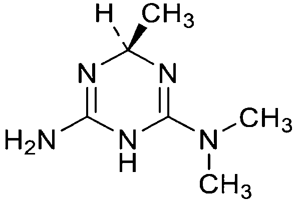

The structure of metformin is shown below.

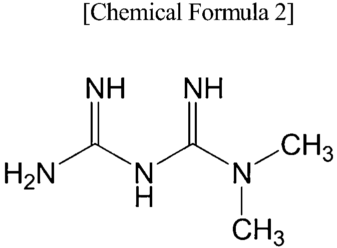

The imeglimin or metformin contained in the ATP secretion inhibitor may be any salt, such as a hydrochloride, an acetate, or a sulfate, as long as it exhibits an ATP secretion inhibitory effect in purinergic chemical transmission. For example, as shown in the following Examples, imeglimin hydrochloride and metformin hydrochloride exhibit an ATP secretion inhibitory effect in purinergic chemical transmission.

Imeglimin hydrochloride was launched in Japan in 2022 as a therapeutic agent for type 2 diabetes. However, since imeglimin shows extremely low transfer into the body, administration of as much as 1 g of imeglimin hydrochloride per day is required. Administration of 1 g of imeglimin hydrochloride daily for one week has been found to result in a blood imeglimin concentration reaching 9 µM. On the other hand, as shown in the following Examples, 0.1 µM to 10 µM imeglimin hydrochloride or metformin hydrochloride causes inhibition of ATP secretion inhibition in purinergic chemical transmission. Accordingly, an ATP secretion inhibitor may be administered to achieve a blood concentration of imeglimin or metformin of 0.05 to 10 µM, preferably 0.1 to 5 µM.

The structure of PEP is shown below.

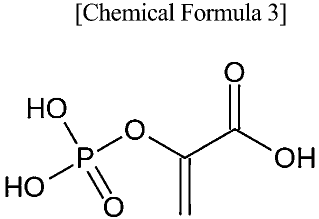

PEP is a glycolytic intermediate metabolite having a phosphate bond that has the highest energy in vivo (-62 KJ/mol). As shown in the Examples below, PEP exhibits an ATP secretion inhibitory effect in purinergic chemical transmission. Furthermore, as shown in the Examples below, L-2-phosphoglyceric acid, D-2-phosphoglyceric acid, and 3-phosphoglyceric acid, which are analogs of PEP, also exhibit an ATP transport inhibitory effect in purinergic chemical transmission similarly to PEP. Accordingly, the ATP secretion inhibitor may include PEP or an analog thereof. The "analog" is the same compound as PEP except that structural modification was made at at least one position. For example, the analog of PEP is the same compound as PEP except that a functional group or an atom has been added, that a functional group or an atom has been removed, or that a functional group or an atom has been replaced with a different functional group or atom. Examples of the PEP analog include L-2-phosphoglyceric acid, D-2-phosphoglyceric acid, and 3-phosphoglyceric acid. PEP or the analog thereof may be a salt such as potassium salt or sodium salt as long as it exhibits an ATP secretion inhibitory effect in purinergic chemical transmission.

Although PEP is present in cells only at an extremely low concentration (in the order of µM), it can freely permeate the cell membrane. Therefore, PEP can be incorporated into cells at an arbitrary concentration.

In cases where the ATP secretion inhibitor includes PEP, the ATP secretion inhibitor may be administered to achieve a blood PEP concentration of 1 to 20 mM, preferably 3 to 10 mM. In cases where the ATP secretion inhibitor includes a PEP analog, the ATP secretion inhibitor may be administered to achieve a blood PEP analog concentration of 1 to 20 mM, preferably 3 to 10 mM.

The ATP secretion inhibitor according to the present embodiment may be a pharmaceutical, an oral composition, or a cosmetic, and may include, in addition to imeglimin, metformin, PEP, an analog of PEP, or a pharmaceutically acceptable salt thereof, other pharmaceutically acceptable components as long as the inhibition of ATP secretion in purinergic chemical transmission is not inhibited.

The form of the ATP secretion inhibitor according to the present embodiment is not particularly limited. Examples of the form of the ATP secretion inhibitor include creams, lotions, gels, ointments, plasters, solutions, dispersions, tablets, fine granules, powders, tablets, capsules. The ATP secretion inhibitor may be an injection, an oral preparation, a topical preparation, a rectal suppository, a vaginal suppository, a preparation for transnasal absorption, a preparation for transdermal absorption, a preparation for transpulmonary absorption, or a preparation for intraoral absorption. Preferably, the ATP secretion inhibitor includes, in addition to imeglimin, metformin, PEP, an analog of PEP, or a pharmaceutically acceptable salt thereof, a pharmaceutically acceptable carrier. The pharmaceutically acceptable carrier includes various organic carrier substances or inorganic carrier substances. Examples of the pharmaceutically acceptable carrier include, for solid preparations, excipients, lubricants, binders, and disintegrants; and, for liquid preparations, solvents, solubilizers, suspending agents, isotonic agents, buffers, and soothing agents. Further, when necessary, additives such as preservatives, antioxidants, colorants, and sweeteners may be used.

Examples of the excipients include lactose, sucrose, D-mannitol, starch, crystalline cellulose, and light anhydrous silicic acid. Examples of the lubricants include magnesium stearate, calcium stearate, talc, and colloidal silica. Examples of the binders include tragacanth gum, acacia, cornstarch, gelatin, crystalline cellulose, sucrose, D-mannitol, dextrin, hydroxypropyl cellulose, hydroxypropyl methylcellulose, and polyvinylpyrrolidone. Examples of the disintegrants include starch, carboxymethyl cellulose, carboxymethyl cellulose calcium, croscarmellose sodium, and carboxymethyl starch sodium.

Examples of the solvents include water for injection, alcohol, propylene glycol, and macrogol. Examples of the solubilizers include polyethylene glycol, propylene glycol, D-mannitol, benzyl benzoate, ethanol, tris(hydroxymethyl)aminomethane, cholesterol, triethanolamine, sodium carbonate, and sodium citrate. The suspending agents include surfactants and hydrophilic polymers, and examples thereof include stearyl triethanolamine, sodium lauryl sulfate, lauryl aminopropionic acid, lecithin, benzalkonium chloride, benzethonium chloride, glyceryl monostearate, polyvinyl alcohol, polyvinylpyrrolidone, sodium carboxymethyl cellulose, methyl cellulose, hydroxymethyl cellulose, hydroxyethyl cellulose, and hydroxypropyl cellulose.

Examples of the isotonic agents include sodium chloride, glycerin, and D-mannitol. Examples of the buffers include buffer solutions of phosphate, acetate, carbonate, and citrate. Examples of the soothing agents include benzyl alcohol. Examples of the preservatives include para-hydroxybenzoate, chlorobutanol, benzyl alcohol, phenethyl alcohol, dehydroacetic acid, and sorbic acid. Examples of the antioxidants include sulfites and ascorbic acid.

The ATP secretion inhibitor according to the present embodiment inhibits ATP secretion in purinergic chemical transmission, and hence can be used for prevention, amelioration, or treatment of a disease or symptom related to purinergic chemical transmission. Examples of the disease or symptom related to purinergic chemical transmission include inflammatory pain, neuropathic pain, ulcerative colitis, non-alcoholic hepatitis, fibrosis of hepatocytes, type 2 diabetes, chronic inflammation, blood coagulation disorder, lower urinary tract dysfunction, pruritus caused by chemical substances or bacterial infection, Parkinson's disease, Alzheimer's disease, temper, atopic dermatitis, hepatic fibrosis, NASH, psoriasis, atherosclerosis, cancer, refractory dermatitis, refractory pain, and melanin deposition.

The ATP secretion inhibitor according to the present embodiment may be for prevention, amelioration, or treatment of the above diseases or symptoms related to purinergic chemical transmission, or may be administered to subjects having these diseases or symptoms. The subjects to which the ATP secretion inhibitor according to the present embodiment are administered are humans and non-human animals. The non-human animals are preferably mammals, more specifically, for example, cats, dogs, cows, pigs, horses, sheep, and deer.

The dose of the ATP secretion inhibitor according to the present embodiment is appropriately determined according to the sex, age, body weight, symptoms, and the like of the subject to whom the ATP secretion inhibitor is administered. The ATP secretion inhibitor is administered such that a therapeutically effective amount of imeglimin, metformin, PEP, an analog of PEP, or a pharmaceutically acceptable salt thereof is achieved. The effective amount is an amount of imeglimin, metformin, PEP, an analog of PEP, or a pharmaceutically acceptable salt thereof that is required to achieve a desired result, in other words, an amount necessary to delay, inhibit, prevent, ameliorate, reverse, or cure the progression of the condition to be treated or managed. The ATP secretion inhibitor may be administered once daily, or in divided doses more than once per day. The ATP secretion inhibitor may be administered at various frequencies. For example, it may be administered daily, every other day, once weekly, every other week, or once monthly.

As shown in the Examples below, the ATP secretion inhibitor according to the present embodiment inhibits activation of hepatic stellate cells (HSCs) or deactivates HSCs. Accordingly, imeglimin, metformin, PEP, an analog of PEP, or a pharmaceutically acceptable salt thereof is useful as a hepatic stellate cell activation inhibitor and a hepatic stellate cell deactivator. Thus, in another embodiment, a hepatic stellate cell activation inhibitor and a hepatic stellate cell deactivator including imeglimin, metformin, PEP, an analog of PEP, or a pharmaceutically acceptable salt thereof are provided.

As shown in the Examples below, the ATP secretion inhibitor according to the present embodiment is effective for treatment of hepatic fibrosis and NASH. Accordingly, imeglimin, metformin, PEP, an analog of PEP, or a pharmaceutically acceptable salt thereof is useful as a hepatic fibrosis therapeutic agent and a NASH therapeutic agent. Thus, another embodiment provides a hepatic fibrosis therapeutic agent and a NASH therapeutic agent including imeglimin, metformin, PEP, an analog of PEP, or a pharmaceutically acceptable salt thereof. The hepatic fibrosis therapeutic agent may be used for prevention of hepatic fibrosis, and the NASH therapeutic agent may be used for prevention of NASH.

Furthermore, as shown in the Examples below, the ATP secretion inhibitor according to the present embodiment is effective for treatment of, and suppression of relapse of, psoriasis. Accordingly, imeglimin, metformin, PEP, an analog of PEP, or a pharmaceutically acceptable salt thereof is useful as a psoriasis therapeutic agent. Thus, another embodiment provides a psoriasis therapeutic agent including imeglimin, metformin, PEP, an analog of PEP, or a pharmaceutically acceptable salt thereof. The psoriasis therapeutic agent may also be used for prevention of psoriasis, or prevention of relapse of psoriasis.

Another embodiment provides use of imeglimin, metformin, PEP, an analog of PEP, or a pharmaceutically acceptable salt thereof for the production of an ATP secretion inhibitor, a hepatic stellate cell activation inhibitor, a hepatic stellate cell deactivator, a hepatic fibrosis therapeutic agent, a NASH therapeutic agent, or a psoriasis therapeutic agent. Another embodiment provides a method of inhibiting ATP secretion. The method of inhibiting ATP secretion includes a step of administering imeglimin, metformin, PEP, an analog of PEP, or a pharmaceutically acceptable salt thereof to a subject, particularly a subject with a disease or symptom related to purinergic chemical transmission. Another embodiment provides imeglimin, metformin, PEP, an analog of PEP, or a pharmaceutically acceptable salt thereof for use in treatment of a disease or symptom related to purinergic chemical transmission.

Another embodiment provides a method of inhibiting hepatic stellate cell activation, a method of deactivating hepatic stellate cells, a method of treating hepatic fibrosis, a method of treating NASH, and a method of treating psoriasis. The method of inhibiting hepatic stellate cell activation, the method of deactivating hepatic stellate cells, the method of treating hepatic fibrosis, the method of treating NASH, and the method of treating psoriasis include a step of administering imeglimin, metformin, PEP, an analog of PEP, or a pharmaceutically acceptable salt thereof to a subject. Another embodiment provides imeglimin, metformin, PEP, an analog of PEP, or a pharmaceutically acceptable salt thereof for use in treatment of hepatic fibrosis, NASH, and psoriasis.

Another embodiment provides use of imeglimin, metformin, PEP, an analog of PEP, or a pharmaceutically acceptable salt thereof for use in inhibition of ATP secretion, inhibition of hepatic stellate cell activation, deactivation of hepatic stellate cells, treatment of hepatic fibrosis, treatment of NASH, or treatment of psoriasis.

### Examples

In the following Examples, unless otherwise specified, sodium clodronate was used as "clodronate"; imeglimin hydrochloride was used as "imeglimin"; and metformin hydrochloride was used as "metformin".

### (Example 1 ATP secretion inhibitory effect of clodronate)

Epidermal keratinocytes are the major source of eATP in the skin. Similar to neuronal cells and hormone-secreting cells, epidermal keratinocytes accumulate ATP in secretory vesicles through the actions of VNUT and V-ATPase, and release ATP by exocytosis in response to ultraviolet (UV), mechanical stimulation, or chemical stimulation. Clodronate is a substance that is known to be a VNUT inhibitor.

### [Culture of Human Epidermal Keratinocytes]

Human epidermal keratinocytes (Cryopreserved HEK, Lot 1758, Cell Applications Inc.) were cultured using an Epivita Keratinocyte Growth Medium Kit according to the manufacturer's instructions. The procedure was as follows. A supplement medium was added to a hydrocortisone-containing basal medium included in the medium preparation kit to prepare a complete medium. The medium in the dish was removed by aspiration. The cells were washed with 5 ml of BSS (Gbco), and then the BSS was removed by aspiration. After addition of 5 to 6 ml of trypsin/EDTA solution (Gbco), the dish was incubated at 37°C for 2 minutes. After addition of 5 ml of trypsin neutralization solution, the resulting cell suspension was transferred into two 15-ml Falcon tubes, and centrifuged at 200 × g for 5 minutes. The supernatant was removed by aspiration, and the pelleted cell cluster was suspended in 2 ml of the medium at 4.5 × 10⁵ cells/ml. An aliquot of the resulting cell suspension was cryopreserved, while another aliquot was cultured and subjected to measurement.

### [Measurement of ATP Secretion]

ATP secretion by human epidermal keratinocytes (hereinafter referred to as "keratinocytes") was measured. Regarding control solutions, Krebs-Ringer HEPES supplemented with 0.2% BSA and 25 mM glucose (hereinafter also referred to as "Krebs/Iono(-)") was used as a negative control, and a solution prepared by adding 5 µM ionomycin to this solution (hereinafter also referred to as "Krebs/Iono(+)") was used as a positive control for ATP secretion. Ionomycin is a Ca²⁺ ionophore. Addition of ionomycin to cells is known to promote influx of extracellular Ca²⁺ into the cells to increase the intracellular Ca²⁺ concentration, resulting in enhancement of ATP secretion. Test solutions were prepared by adding clodronate to Krebs/Iono(-) or Krebs/Iono(+) to a final concentration of 0.1 µM (hereinafter also referred to as "Clo/Iono(-)" or "Clo/Iono(+)", respectively) (N = 3).

Keratinocytes were cultured at 2.5 × 10⁴ cells/well in a 24-well plate at 37°C in 5% CO₂ for 4 days. The medium was removed by aspiration, replaced with Krebs/Iono(-) or Clo/Iono(-), and incubated at 37°C for 6 hours. These solutions were then removed by aspiration, and the content of each well incubated with Krebs/Iono(-) was replaced with 300 µl of a control solution (Krebs/Iono(-) or Krebs/Iono(+)), while the content of each well incubated with Clo/Iono(-) was replaced with 300 µl of a test solution (Clo/Iono(-) or Clo/Iono(+)). After further incubation at 37°C for 20 minutes, 200 µl of the supernatant was collected. The collected supernatant was immediately treated on a heat block at 95°C for 10 minutes, to inactivate ectonucleotidase. ATP in the supernatant was quantified using a Dojindo ATP assay kit - Luminescence (manufactured by Dojindo Laboratories).

### (Results)

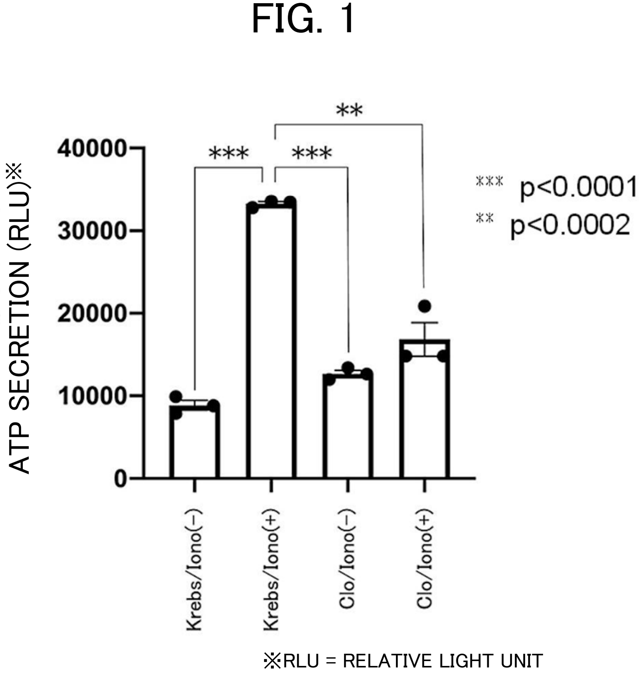

As shown in FIG. 1, ATP secretion increased in Krebs/Iono(+). On the other hand, the ATP secretion level in Clo/Iono(+) was almost the same as those in Krebs/Iono(-) and Clo/Iono(-). These results showed that ionomycin promotes ATP secretion in keratinocytes and that clodronate, a known VNUT inhibitor, inhibits the ATP secretion promoted by ionomycin. Thus, these data support the validity of use of keratinocytes to study the ATP secretion inhibitory effect.

### (Example 2 ATP secretion inhibitory effect of imeglimin)

Keratinocytes were cultured in the same manner as in Example 1, and ATP secretion from the keratinocytes was measured. Krebs/Iono(-), or a solution prepared by adding 10 µM imeglimin, 0.1 µM eicosapentaenoic acid (EPA), 1 µM EPA, or 10 µM clodronate to Krebs/Iono(-) was used (N = 4). Similarly to clodronate, EPA is a substance known to have an ATP secretion inhibitory action.

After culturing keratinocytes, the medium was removed by aspiration, and replaced with Krebs/Iono(-), or Krebs/Iono(-) supplemented with 10 µM imeglimin, 0.1 µM EPA, 1 µM EPA, or 10 µM clodronate, followed by incubation for 6 hours. Subsequently, the solution was replaced with either a control solution (Krebs/Iono(-) or Krebs/Iono(+)) or a test solution (Krebs/Iono(+) supplemented with 10 µM imeglimin, 0.1 µM EPA, 1 µM EPA, or 10 µM clodronate), followed by incubation at 37°C for 20 minutes and then collection of 200 µl of the supernatant. The collected supernatant was immediately treated on a heat block at 95°C for 10 minutes to inactivate ectonucleotidase. ATP in the supernatant was quantified using a Dojindo ATP assay kit - Luminescence (manufactured by Dojindo Laboratories).

### (Results)

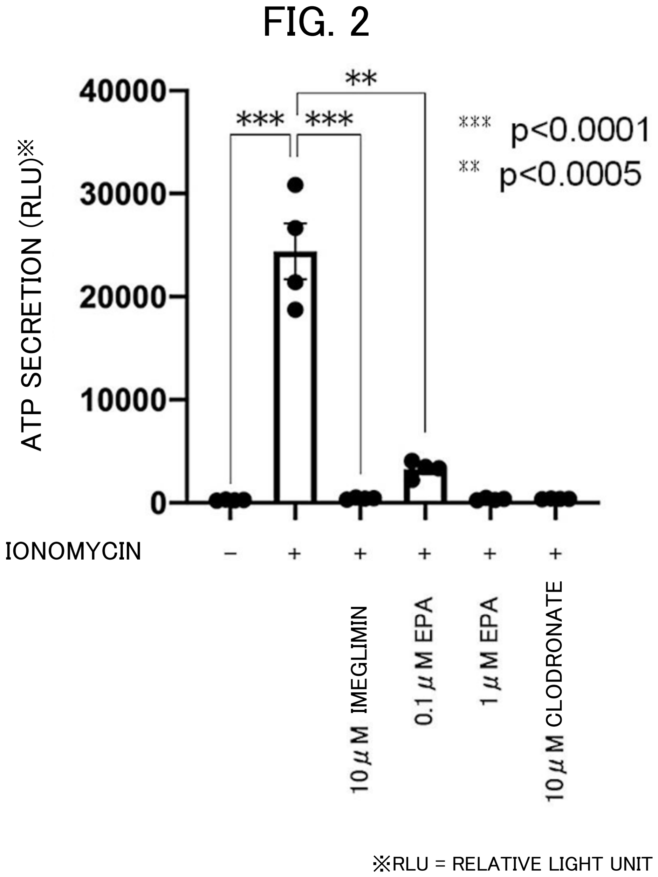

As illustrated in FIG. 2, ATP secretion promoted by ionomycin was shown to be inhibited in all test solutions. The ATP secretion inhibitory effect of 10 µM imeglimin was shown to be comparable to those of 1 µM EPA and 10 µM clodronate. The viability of keratinocytes in the present Example was 79%, and no difference in the viability was found between the cells treated with the controls and the cells treated with the test solutions.

### (Example 3 ATP secretion inhibitory effects of imeglimin and metformin at various concentrations)

Keratinocytes were cultured in the same manner as in Example 1, and the medium was replaced with Krebs/Iono(-), Krebs/Iono(-) supplemented with 0.1 µM, 1 µM, or 10 µM imeglimin, or Krebs/Iono(-) supplemented with 0.1 µM, 1 µM, or 10 µM metformin, followed by incubation for 6 hours. Subsequently, the solution was replaced with either a control solution (Krebs/Iono(-) or Krebs/Iono(+)) or a test solution (Krebs/Iono(+) supplemented with 0.1 µM, 1 µM, or 10 µM imeglimin, or Krebs/Iono(+) supplemented with 0.1 µM, 1 µM, or 10 µM metformin), followed by incubation at 37°C for 20 minutes and then collection of 200 µl of the supernatant. The collected supernatant was immediately treated on a heat block at 95°C for 10 minutes to inactivate ectonucleotidase. ATP in the supernatant was quantified using a Dojindo ATP assay kit - Luminescence (manufactured by Dojindo Laboratories) (N = 3).

### (Results)

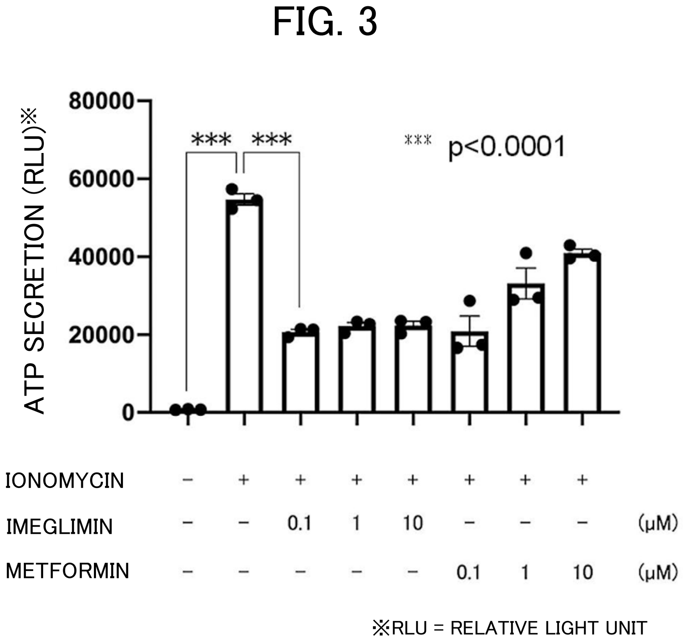

As shown in FIG. 3, imeglimin tended to show a slightly higher ATP secretion inhibitory effect than metformin. However, both imeglimin and metformin were shown to exhibit an ATP secretion inhibitory effect even at a concentration of as low as 0.1 µM. Metformin showed a decreased inhibitory effect at higher concentrations.

### (Example 4 Study of incubation time for ATP secretion inhibitory effect of imeglimin)

Keratinocytes were cultured in the same manner as in Example 1, and the medium was replaced with Krebs/Iono(-), or Krebs/Iono(-) supplemented with 1 µM imeglimin, followed by incubation for 2 hours or 6 hours. Subsequently, the solution was replaced with either a control solution (Krebs/Iono(-) or Krebs/Iono(+)) or a test solution (Krebs/Iono(+) supplemented with 1 µM imeglimin), followed by incubation at 37°C for 20 minutes and then collection of 200 µl of the supernatant. The collected supernatant was immediately treated on a heat block at 95°C for 10 minutes to inactivate ectonucleotidase. ATP in the supernatant was quantified using a Dojindo ATP assay kit - Luminescence (manufactured by Dojindo Laboratories) (N = 3).

### (Results)

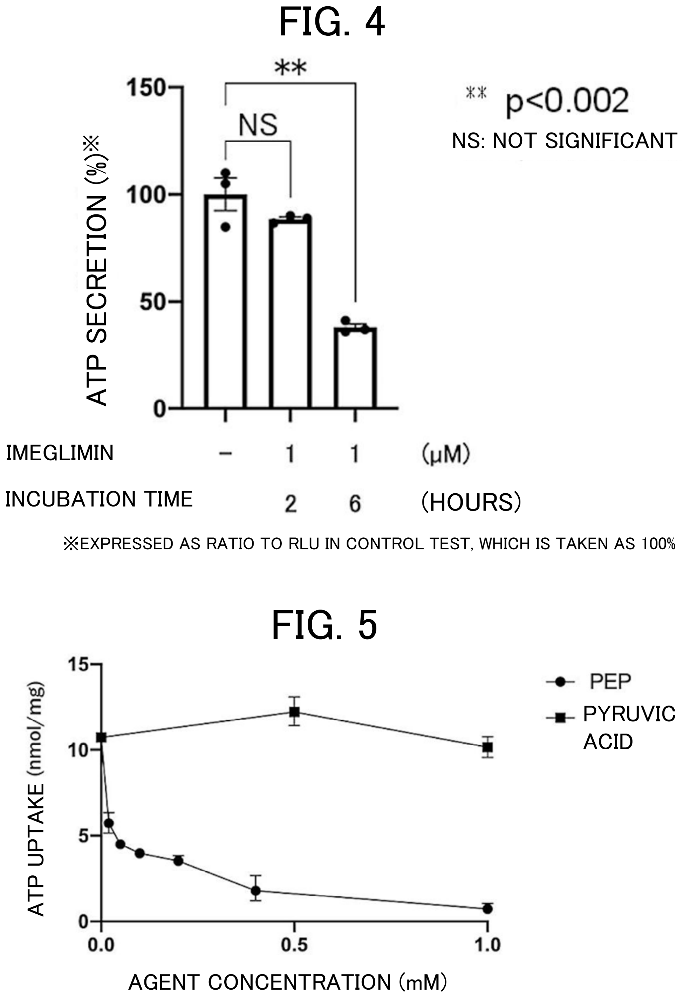

FIG. 4 illustrates the ratio of the relative light unit (RLU) value corresponding to the ATP secretion level obtained using the test solution, to the RLU value corresponding to the ATP secretion level obtained using the control solution. In the case where the incubation time after the replacement of the medium with the test solution was 2 hours, the ATP secretion level was about 85% of that obtained using the control solution, indicating no marked ATP secretion inhibitory effect. On the other hand, In the case where the incubation time was 6 hours, the ATP secretion level was about 40% of that obtained using the control solution, indicating a pronounced ATP secretion inhibitory effect. These results indicated that the ATP secretion inhibitory effect of imeglimin does not become maximal immediately after addition of imeglimin to cells and that production of a strong inhibitory effect by imeglimin requires about 6 hours.

### (Example 5 ATP transport inhibitory action in the chromaffin granule membrane)

Chromaffin granules in the adrenal medulla are representative ATP-loaded granules, and contain about 0.1 M ATP. Stimulation of chromaffin cells causes release of catecholamines and ATP from the inside of the chromaffin granules. It has long been known that uptake of ATP into isolated chromaffin granules is inhibited by 1 to 10 mM PEP. Whether ATP transport activity of the chromaffin granule membrane is mediated by VNUT remains unclear. In view of this, first, the action of PEP on the ATP transport activity in isolated chromaffin granule membranes was investigated.

### [Preparation of chromaffin granule membrane vesicles]

Chromaffin granule membranes were prepared from adrenal glands collected from slaughtered cattle according to Reference A (Nathan Nelson et al., 1988, Chromaffin Granule Proton Pump, Methods in Enzymology, 157, 619-633). Chromaffin granule membranes prepared by sucrose density gradient centrifugation were suspended in 2 ml of SME buffer (20 mM MOPS-tris (pH 7.0), 0.3 M sucrose, 5 mM EDTA, 10 µg/ml leupeptin, and 10 µg/ml pepstatin) + 25% glycerol, and the resulting suspension was aliquoted and stored at -80°C. Under these conditions, the ATP transport activity, the V-ATPase activity, and the catecholamine transport activity remain fully active for at least one year.

An ATP transport inhibition experiment for the chromaffin granule membrane was carried out according to Reference B (Yoshinori Moriyama et al., 2022, Is the vesicular nucleotide transporter a molecular target of eicosapentaenoic acid?, Frontiers in Pharmacology, 103389). About 20 µg of chromaffin granule membranes were added to 0.5 ml of measurement buffer (20 mM MOPS-tris (pH 7.0), 0.3 M sucrose, 5 mM potassium chloride, 5 mM magnesium acetate, 5 mM creatine phosphate, 10 units of creatine kinase, and 1 mM ³[H]-ATP [15 KBq/assay]), followed by incubation at 30°C for 1 hour. Through a Millipore MCE membrane filter with a pore size 0.45 µm, 0.2 ml of the reaction liquid was filtered by suction. Thereafter, the filter was washed with 10 ml of 20 mM MOPS-tris (pH 7.0), 0.3 M sucrose, 5 mM potassium chloride, and 5 mM magnesium acetate, and dissolved in 10 ml of Clear-sol II (manufactured by Nacalai Tesque, Inc.). The radioactivity was then measured using a liquid scintillation counter (N = 3).

The reaction was initiated by adding 50 mM ³[H]-ATP to a final concentration of 1 mM. The 50 mM ³[H]-ATP was prepared by mixing an ATP-tris solution (manufactured by Sigma-Aldrich) with [2,8-³H] adenosine triphosphate tetrasodium salt (26.0 Ci/mmol) (manufactured by New England Nuclear). PEP was used as a test reagent, and pyruvic acid was used as a negative control. Five minutes before the addition of ATP, PEP or pyruvic acid, as a solution in 20 mM MOPS-tris (pH 7.0), 0.3 M sucrose, 5 mM potassium chloride, and 5 mM magnesium acetate, was added to the measurement liquid to a final PEP concentration of 0.02, 0.05, 0.1, 0.2, 0.4, or 1 mM, or to a final pyruvic acid concentration of 0.5 or 1 mM.

A catecholamine transport inhibition experiment was carried out in the same manner as the ATP transport experiment except that the experiment used a measurement liquid containing 20 mM MOPS-tris (pH 7.0), 0.1 M sucrose, 0.1 M potassium chloride, 5 mM magnesium acetate, and about 5 µg of chromaffin granule membranes. To 0.5 ml of the measurement liquid, 2 mM ATP was added, and the resulting mixture was incubated at 30°C for about 5 minutes, followed by addition of³ [H]-dopamine thereto to a final concentration of 10 µM. After further incubation at 30°C for 15 minutes, 0.2 ml of the reaction liquid was subjected to suction filtration through a Millipore MCE membrane filter with a pore size of 0.45 µm, and the radioactivity on the filter was measured (N = 3). The substrate, ³[H]-dopamine, was prepared by mixing 3,4-[RING-2,5,6-³H]dihydroxyphenylethylamine (dopamine) (30 Ci/mmol) (manufactured by New England Nuclear) with dopamine hydrochloride (manufactured by LKT Laboratories) such that a final concentration of 0.1 mM was achieved during the use.

ATP-dependent proton transport by V-ATPase was measured according to Reference B. Proton transport activity was measured using a spectrofluorometer at excitation wavelength of 492 nm and a fluorescence wavelength 540 nm, wherein fluorescence quenching of acridine orange was used as an index (N = 2). The transmembrane potential generated was measured using a spectrofluorometer at an excitation wavelength of 580 nm and a fluorescence wavelength of 630 nm, wherein fluorescence quenching of oxonol-V was used as an index (N = 2). The acridine measurement liquid (2 ml) contained 20 mM MOPS-tris (pH 7.0), 0.2 M sucrose, 0.1 M potassium chloride, 5 mM magnesium acetate, 0.2 µg valinomycin, 2 µM acridine orange, and about 20 µg of chromaffin granules. The oxonol-V measurement liquid (2 ml) contained 20 mM MOPS-tris (pH 7.0), 0.3 M sucrose, 5 mM magnesium acetate, 5 µM oxonol-V, and about 20 µg of chromaffin granules.

### (Results)

FIG. 5 illustrates ATP transport activity as the ATP uptake per mg protein. As illustrated in FIG. 5, PEP inhibited ATP transport with an IC₅₀ (concentration at 50% inhibition) of 25 µM, whereas pyruvic acid did not inhibit ATP transport at the concentrations of up to 1 mM.

On the other hand, PEP did not inhibit V-ATPase or catecholamine transport at the concentrations of up to 5 mM (data not shown). Specifically, the ATP-dependent dopamine transport activity in the control test was (11.07 ± 0.129) nmol/mg protein, and the ATP-dependent dopamine transport activity in the presence of 5 mM PEP was (12.41 ± 0.250) nmol/mg protein (N = 3). It has been confirmed that ATP-dependent dopamine transport is almost completely blocked by 1 µM reserpine and exhibits characteristics of typical vesicular catecholamine transport.

The addition of ATP quenched the acridine orange fluorescence by 62% (relative to the fluorescence intensity before the addition of ATP, which was taken as 100). The addition of 5 mM PEP did not change the level of quenching (N = 2). The addition of ATP quenched the oxonol-V fluorescence by 40% (relative to the fluorescence intensity before the addition of ATP, which was taken as 100). The addition of 5 mM PEP did not change the level of quenching (N = 2).

In addition to PEP, PEP analogs (L-2-phosphoglyceric acid, D-2-phosphoglyceric acid, and 3-phosphoglyceric acid) showed an ATP transport inhibitory action. Their IC₅₀ values were 0.18, 0.18, and 0.45 mM, respectively. On the other hand, no inhibitory activity was found with glucose-6-phosphate, fructose-6-phosphate, pyruvic acid (see FIG. 5), L-lactic acid, malic acid, oxaloacetic acid, citric acid and other TCA cycle intermediate metabolites, acetyl-CoA, or NAD+.

### (Example 6 Human VNUT inhibitory action)

Valinomycin enables ATP loading into liposomes containing human VNUT. The inhibitory action of PEP on this ATP loading was tested as follows.

### [Preparation of Human VNUT]

Human VNUT was prepared according to Reference C (Keisuke Sawada et al., 2008, Identification of a vesicular nucleotide transporter, Proc Natl Acad Sci USA, vol. 105, no. 15, 5683-5686). Insect cells (HIGH FIVE cells) were infected at MOI = 1 with a baculovirus in which a human VNUT expression system was incorporated. HIGH FIVE cells expressing VNUT (1 × 10⁸ cells) were collected with a cell scraper, and suspended at about 8 mg/ml in 20 mM Tris-HCl (pH 8.0), 0.1 M potassium acetate, 10% glycerol, 10 µg/ml pepstatin A, and 10 µg/ml leupeptin, followed by homogenization of the cells using an ultrasonic generator (manufactured by Sonics Materials). The cell homogenate was centrifuged at 700 × g for 10 minutes to remove non-homogenized cells, and then ultracentrifugation was performed at 160,000 × g for 1 hour, followed by collecting the cell membrane as a pellet. The pellet was suspended in the same buffer, and 20% octylglucoside was added thereto to a final concentration of 2%. The resulting mixture was vortexed, and left to stand on ice for 30 minutes. This solution was ultracentrifuged again at 160,000 × g for 1 hour, and the supernatant, containing solubilized VNUT, was collected as a crude VNUT fraction.

### [Preparation of VNUT-Containing Reconstituted Liposomes]

A soybean phospholipid liposome solution was prepared as follows. After adding 0.5 g of Type II L-α-phospholipids from soybean (manufactured by Sigma) to 25 ml of 20 mM MOPS-tris (pH 7.0) and 1 mM dithiothreitol, the resulting mixture was sonicated using an ultrasonic generator (manufactured by Sonics Materials) to achieve homogenization. The resulting homogenate was aliquoted and stored at -80°C.

VNUT (protein, 20 µg) prepared by the above method was mixed with liposomes (lipid weight, 200 µg), and left to stand at -80°C for 30 minutes. The mixture was then rapidly thawed at 37°C then and diluted with 20 volumes of 20 mM MOPS-Tris (pH 7.0), 0.15 M sodium acetate, and 5 mM magnesium acetate. The resulting dilution was left to stand on ice for 10 minutes, and then centrifuged at 160,000 × g for 1 hour at 4°C, to obtain a clear pellet (reconstituted liposomes). The reconstituted liposomes were suspended in 20 mM MOPS-Tris (pH 7.0), 0.15 M sodium acetate, and 5 mM magnesium acetate, and then left to stand on ice. The reconstituted liposomes obtained were subjected to SDS-polyacrylamide gel electrophoresis using an ePAGEL E-T520 2-10% gradient gel (manufactured by Atto) and then to Coomassie Brilliant Blue staining. A major band was detected at a position of about 68 kDa. The proteins after the electrophoresis with the ePAGEL E-T520 2-10% gradient gel (manufactured by Atto) were electrotransferred to a nitrocellulose sheet, and analyzed by Western blotting using an anti-human SLC17A9 (VNUT) rabbit polyclonal antibody (manufactured by Proteintech). The results showed that the band described above was a VNUT antibody-positive band and that almost the entire amount of the VNUT added was collected into the reconstituted liposomes.

### [Measurement of ATP Transport Activity]

The ATP transport activity of the VNUT-containing reconstituted liposomes prepared by the above method was measured. The measurement liquid contained 0.5 ml of 20 mM MOPS-Tris (pH 7.0), 0.1 M potassium acetate, 10 mM potassium chloride, 5 mM magnesium acetate, 0.1 µg valinomycin (added after being prepared as a 0.1 mg/ml solution in ethanol), and 0.1 mM radiolabeled ³H-ATP (15 KBq/assay). As a test reagent, 0.1 mM PEP was used. As a negative control for inhibition of ATP loading, 2 mM pyruvic acid was used. As a positive control for inhibition of ATP loading, 1 µM carbonyl cyanide-m-chlorophenyl hydrazone (carbonyl cyanide 3-chlorophenyl hydrazone; CCCP) was used. CCCP is known to inhibit valinomycin-dependent ATP loading in human VNUT. PEP or pyruvic acid was dissolved in 20 mM MOPS-Tris (pH 7.0), 0.1 M potassium acetate, 10 mM potassium chloride, and 5 mM magnesium acetate, and added to the measurement liquid. The reaction was initiated by adding ³H-ATP. The measurement liquid was incubated at 30°C for 2 minutes, and then the entire measurement liquid was filtered through a 0.22-µm Millipore filter. The filter was washed with 10 ml of 20 mM MOPS-Tris (pH 7.0), 0.1 M potassium acetate, 10 mM potassium chloride, and 5 mM magnesium acetate, and the radioactivity retained on the filter was measured with a liquid scintillation counter (N = 3). Ethanol (1 µl) was added in place of valinomycin, to obtain the background corresponding to the absence of membrane potential.

### (Results)

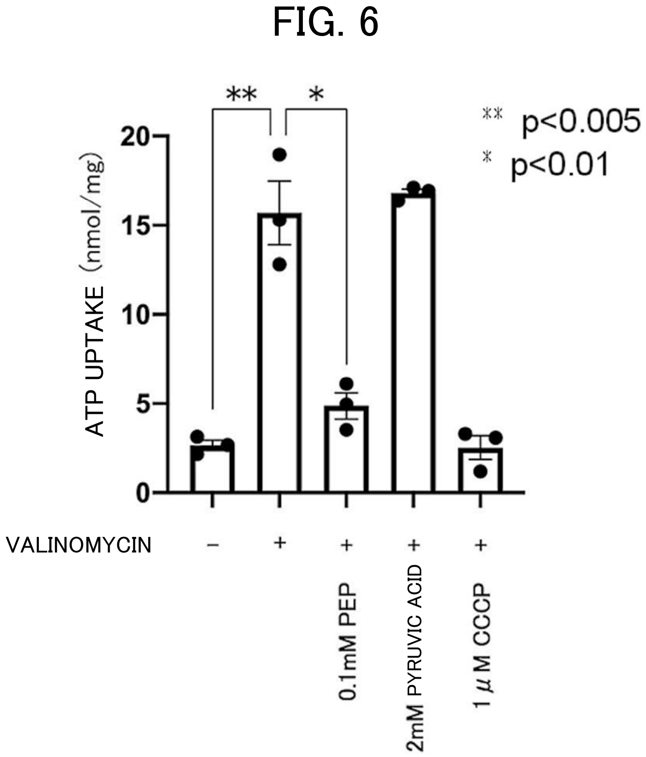

As illustrated in FIG. 6, ATP loading into liposomes was strongly inhibited by 0.1 mM PEP. On the other hand, pyruvic acid at a 50-fold higher concentration (5 mM) did not inhibit ATP loading into liposomes. These findings indicated that PEP has a VNUT inhibitory effect.

### (Example 7 ATP secretion inhibitory effect of PEP in keratinocytes)

According to Example 6, PEP was suggested to have a VNUT inhibitory effect. A further study was carried out regarding the effect of PEP on ATP secretion. Keratinocytes were cultured in the same manner as in Example 1, and ATP secretion from the keratinocytes was measured. However, in the measurement of ATP secretion, the incubation time after the replacement with the control solution or test solution was set to 20 minutes. The test solution used was prepared by adding 5 mM or 10 mM PEP to Krebs/Iono(+) (N = 4).

### (Results)

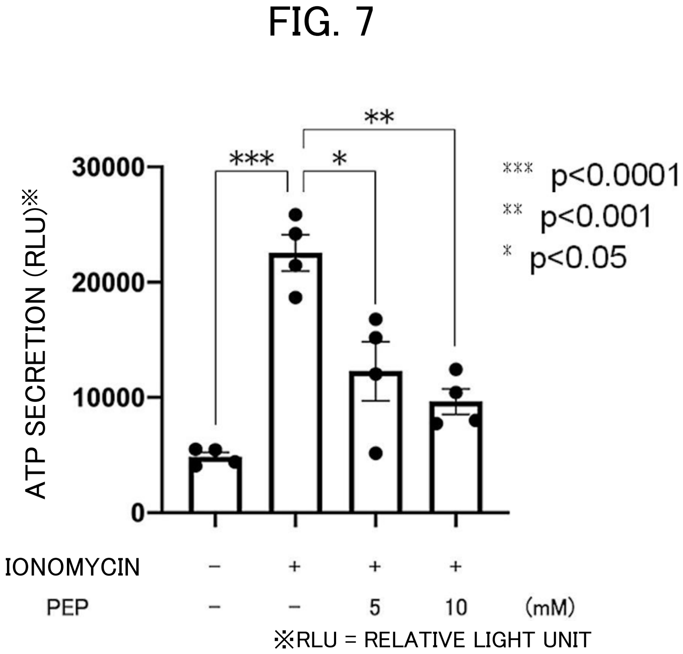

As illustrated in FIG. 7, ATP secretion promoted by ionomycin was shown to be inhibited by 5 mM or 10 mM PEP. Further, PEP at not less than 3 mM exhibited a relatively strong ATP secretion inhibitory effect (data not shown).

In the above Examples and figures, each value is expressed as the mean ± experimental error (SE) for the number of trials indicated by N. A Student's t-test was performed with a significance level of 0.01.

### (Example 8 ATP secretion inhibitory effect of PEP in keratinocytes)

Upon reception of stimuli from the external environment such as bacterial infection and UV, skin tissues cause various immune reactions. Keratinocytes express receptors such as CD4 and Toll-like receptor 4, and binding of lipopolysaccharide (LPS), a constituent of the bacterial outer membrane, to those receptors leads to various immune reactions. Inflammatory reactions caused by LPS stimulation are used in standard methods for evaluation of anti-inflammatory agents. In the present Example, ATP secretion in response to LPS, a physiological stimulant of keratinocytes, was examined.

Keratinocytes were cultured in the same manner as in Example 1. Keratinocytes were cultured at 2.5 × 10⁴ cells/well in a 24-well plate at 37°C in 5% CO₂ for 4 days. The medium was replaced with a test solution (Hanks' balanced salt solution (HBSS) (manufactured by Gibco) supplemented with imeglimin, metformin, or clodronate), and then incubation was performed at 37°C in 5% CO₂ for 18 hours. Thereafter, LPS (10 µg/ml) was added thereto, and incubation was further carried out for 10 minutes, followed by collection of 200 µl of the supernatant. The supernatant was immediately treated on a heat block at 95°C for 10 minutes, and ATP in the supernatant was quantified using an ATP Assay Kit - Luminescence (manufactured by Dojindo) (N = 8).

### (Results)

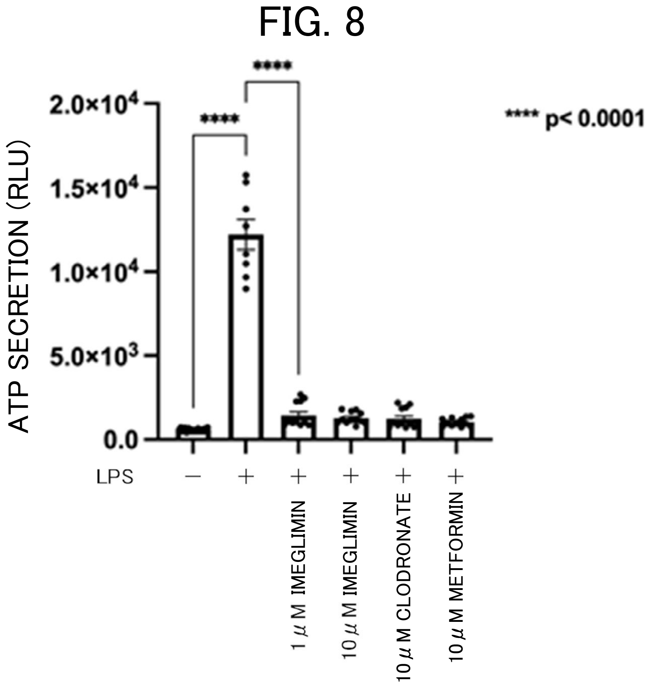

As illustrated in FIG. 8, ATP was secreted from the keratinocytes upon the LPS stimulation. This secretion was almost completely inhibited by 1 µM imeglimin, 10 µM imeglimin, 10 µM metformin, or 10 µM clodronate. An RLU of 1.0 × 10⁴ corresponds to 29 nM ATP. The viability of keratinocytes after the measurement was not less than 99.9%. A marked improvement in the viability of keratinocytes was achieved by the incubation in HBSS.

### (Example 9 ATP secretion inhibitory action of imeglimin in macrophage-like cells)

Macrophages engulf foreign bodies such as bacteria, and secrete cytokines to activate the immune function. Macrophages are cells that secrete ATP to transmit inflammatory signals, and play a major role in innate immunity. The ATP secretion mechanism in macrophages is known to be intravesicular ATP loading by VNUT, and exocytosis (Reference D; Hayato Sakaki, 2013, Autocrine Regulation of Macrophage Activation via Exocytosis of ATP and Activation of P2Y11 Receptor, PLoS One, 8(4):e59778). The ATP secretion inhibitory action of imeglimin was studied in macrophages.

Human acute monocytic leukemia cells (THP-1 cells (ACTT)) were cultured in accordance with Reference D. THP-1 cells were cultured in RPMI1640 medium supplemented with 10% fetal bovine serum (FBS), 100 units/ml penicillin, and 100 µg/ml streptomycin at 37°C in 5% CO₂ for 1 day. Further, THP-1 cells were cultured in RPMI1640 medium supplemented with 1 µg/ml *Escherichia coli* scrotype 055:B5 (manufactured by Sigma-Aldrich) and 1000 units/ml mouse IFN-γ (manufactured by R&D Systems) at 37°C in 5% CO₂ for 1 day, to allow differentiation into macrophages.

The differentiated macrophages were cultured in RPMI1640 medium supplemented with 1 mg/ml *Escherichia coli* scrotype 055:B5 (manufactured by Sigma-Aldrich) and 1000 units/ml mouse IFN-γ (manufactured by R&D Systems) at 2.5 × 10⁴ cells/well in a 24-well plate at 37°C in 5% CO₂ for 4 days. The medium was replaced with HBSS (manufactured by Gibco) supplemented with imeglimin or clodronate), and then LPS (10 µg/ml) was added thereto, followed by further performing incubation for 10 minutes and then collection of 200 µl of the supernatant. The supernatant was immediately treated on a heat block at 95°C for 10 minutes, and ATP in the supernatant was quantified using an ATP assay kit - Luminescence (manufactured by Dojindo) (N = 10).

### (Results)

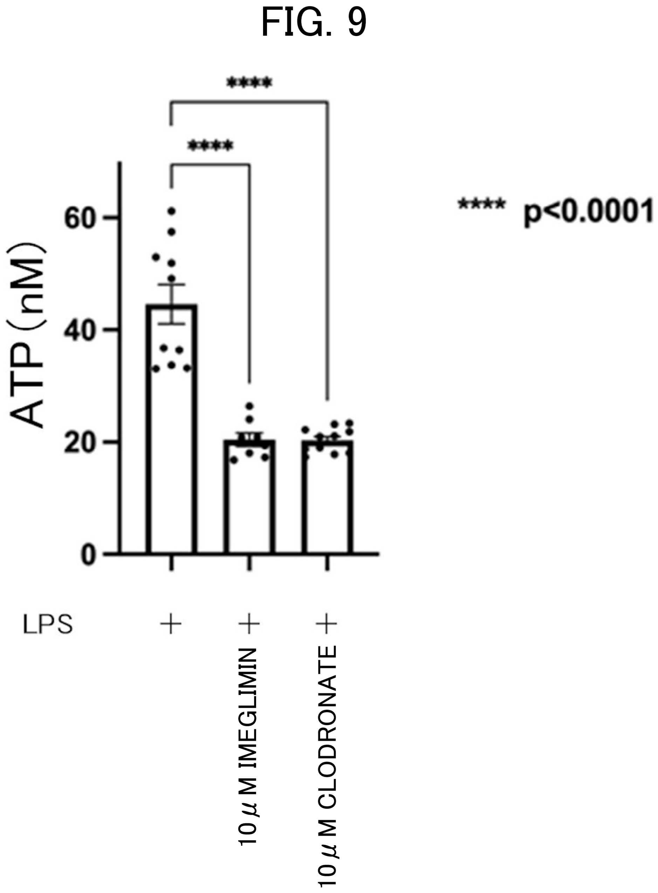

As illustrated in FIG. 9, the LPS stimulation induced secretion of ATP from the macrophages at a level comparable to that reported in Reference D. The amount of ATP secreted was expressed in the same manner as in Reference D. This ATP secretion was strongly inhibited by 10 µM imeglimin or 10 µM clodronate.

### (Example 10 Inhibition of ATP loading into ATP-loaded granules by a fluorescent ATP derivative)

When 2'/3'-O-(*N*- methyl-anthraniloyl)-adenosine-5'-triphosphate triethylammonium salt (MANT-ATP, manufactured by Invitrogen), a fluorescent ATP derivative, is incubated with keratinocytes, it is taken up into the cells and accumulates in ATP-loaded granules. Since inhibition of VNUT expression by RNAi prevents this accumulation, the accumulation has been thought to occur as a result of recognition of MANT-ATP as a substrate by VNUT and its transportation into the granules (Reference E: Kaori Inoue et al., 2014, Mechanism Underlying ATP Release in Human Epidermal Keratinocytes, J. Invest. Dermatol., 134, 1465-1468). ATP-loaded granules in macrophages likewise accumulate MANT-ATP (see Reference D). In order to confirm, by another approach, that MANT-ATP is a transport substrate of VNUT, the effect of MANT-ATP on ATP uptake into bovine adrenal medullary chromaffin granule membrane vesicles, which are ATP-loaded granules, was studied. In this experiment, MANT-ATP is expected to act as a competitive inhibitor for VNUT to inhibit ATP-dependent uptake of radiolabeled ATP.

Chromaffin granule membrane vesicles were prepared in the same manner as in Example 5, and uptake of radiolabeled ATP into the vesicles was measured. Transport activity of catecholamine (dopamine) and ATP-dependent proton transport by V-ATPase were also measured in the same manner as in Example 5.

### (Results)

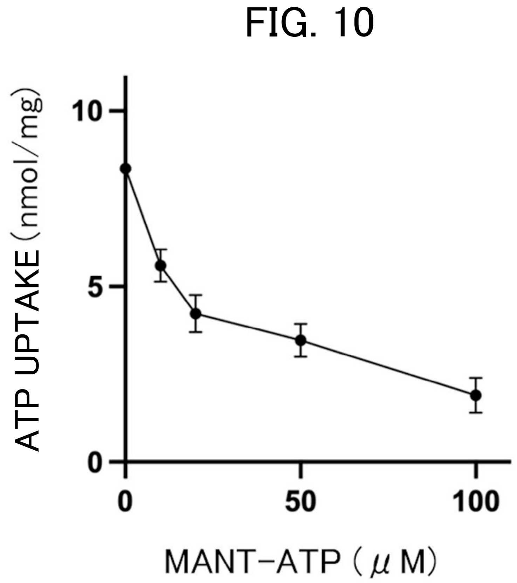

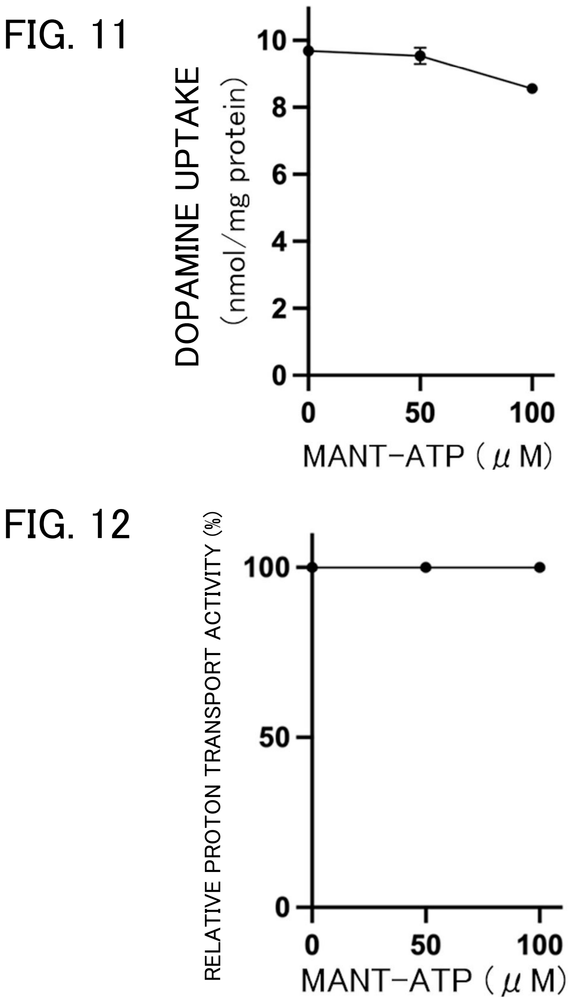

As illustrated in FIG. 10, MANT-ATP strongly inhibited ATP transport (N = 3). The ID₅₀ was about 30 mM. As illustrated in FIG. 11, MANT-ATP hardly affected the catecholamine transport system (N = 3). As illustrated in FIG. 12, MANT-ATP did not affect ATP-dependent proton transport by V-ATPase, which drives ATP transport, at concentrations of up to 100 µM (N = 1). A proton transport activity (relative value, 10%) corresponds to 61.8% quenching of acridine orange fluorescence. V-ATPase can also recognize MANT-ATP as a substrate, hydrolyze MANT-ATP, and transport protons. Upon addition of 0.1mM MANT-ATP, acridine orange fluorescence was quenched by 34%, and subsequent addition of ATP increased total fluorescence quenching to 61.8%. Thus, MANT-ATP does not inhibit V-ATPase. These findings demonstrate that MANT-ATP competitively inhibits ATP transport by VNUT and that MANT-ATP is taken up into ATP-loaded vesicles by VNUT.

### (Example 11 Detection of ATP-loaded granules in keratinocytes using MANT-ATP, and inhibition of MANT-ATP loading by imeglimin)

In order to demonstrate that the target of the ATP secretion inhibitory effect of imeglimin is ATP-loaded vesicles, the effect of imeglimin on intragranular accumulation of ANT-ATP was studied. Keratinocytes were cultured in the same manner as in Example 8. Keratinocytes were cultured at 4 × 10⁴ cells/ml in a glass-bottom dish (35 mm, manufactured by Matsunami Glass Ind., Ltd.) at 37°C in 5% CO₂ for 2 days. The culture medium was then replaced with a medium supplemented with MANT-ATP (manufactured by Invitrogen) at a final concentration of 0.3 mM, and further incubation was performed for 4.5 hours. Subsequently, imeglimin was added thereto to a final concentration of 10 µM, and further incubation was performed at 37°C in 5% CO₂ for 19 hours. Thereafter, the cells were washed twice with HBSS (manufactured by Gibco) and observed using an inverted confocal laser microscope (FV-1000 OSR, manufactured by Olympus Corporation).

### (Results)

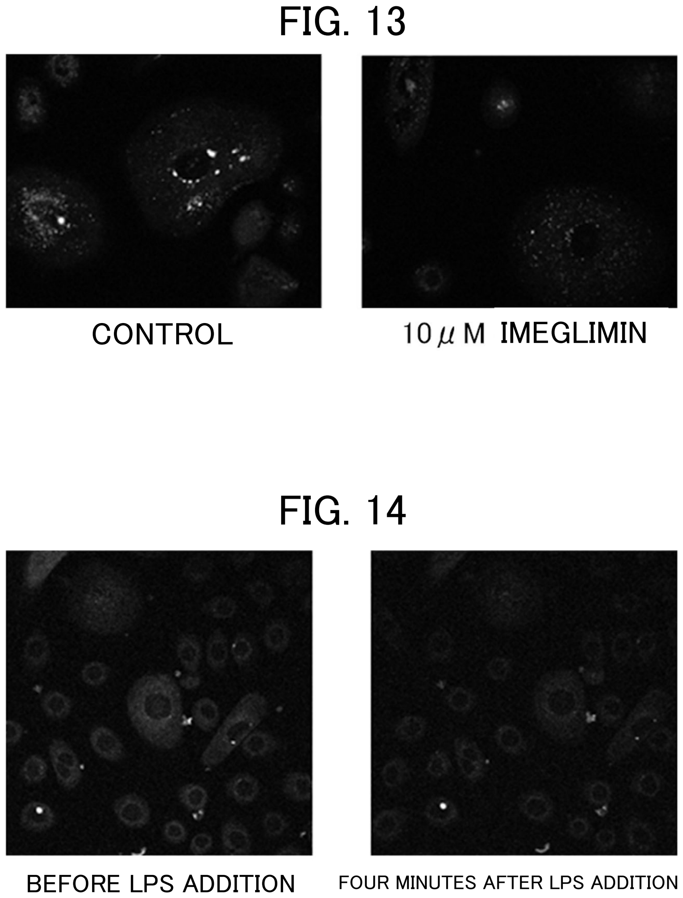

As illustrated in FIG. 13, the control showed vesicles in which MANT-ATP had accumulated, and those vesicles were found mainly around nuclei, indicating accumulation of MANT-ATP in the granules in the keratinocytes. In contract, this accumulation was absent in the cells treated with 10 µM imeglimin. These findings indicated that the target of imeglimin is ATP-loaded vesicles. Based on the results of Example 4 and other experiments, neither imeglimin nor metformin exhibited a strong inhibitory effect on VNUT or V-ATPase (data not shown). Thus, the inhibition of intravesicular ATP loading by imeglimin is thought not to be due to a direct short-term action on VNUT, but is thought to be due to actions resulting from, for example, changes in ion permeability of the ATP-loaded vesicle membrane, changes in the energy state, effects of post-translational modification and the like of VNUT and V-ATPase, and inhibition of VNUT expression.

### (Example 12 Disappearance of intragranular MANT-ATP due to ionomycin or LPS)

As demonstrated in Examples 1 to 4 and 8, ionomycin or LPS can cause release of ATP from keratinocytes. Examples 9 and 10 above demonstrated that the released ATP is derived from ATP loaded in secretory granules. Thus, ionomycin or LPS may also cause extracellular release of MANT-ATP that has accumulated in the granules. Accordingly, this process was observed by confocal microscopy.

In the same manner as in Example 8, keratinocytes were cultured at 2.5×10⁴ cells/well in a 24-well plate at 37°C in 5% CO₂ for 2 days. The culture medium was replaced with a medium supplemented with MANT-ATP (manufactured by Invitrogen) at a final concentration of 0.3mM, and the cells were further incubated at 37°C and 5% CO₂ for 4.5 hours. The cells were washed twice with HBSS (manufactured by Gibco), and the medium was replaced with HBSS, followed by addition of LPS (10µg/ml). Disappearance of MANT-ATP was then observed by confocal microscopy.

### (Results)

As illustrated in FIG. 14, the addition of LPS (10µg/ml) caused rapid disappearance of granular MANT-ATP signals. Addition of 10µM ionomycin yielded similar results. These results indicate that ATP secretion inhibitors such as imeglimin do not affect the process of exocytosis of secretory vesicles.

### (Example 13 Study of VNUT expression in hepatic stellate cells)

According to Reference F (Shanu Jain and Kenneth A. Jacobson, 2021, Purinergic signaling in Liver pathophysiology, Frontiers in Endocrinology, 12, 718429), ATP signaling through purinergic receptors is involved in activation of HSC. Candidate sources of ATP have included hepatocytes, macrophages, and platelets, but the possibility that HSCs themselves secrete ATP has not been considered so far. If HSCs secrete ATP, ATP and its metabolite (adenosine) would be capable of effectively activating HSCs, and hence an ATP secretion inhibitor would be capable of effectively blocking ATP signaling to allow effective inhibition of HSC activation and induction of HSC deactivation. In view of this, the present Example and Example 14 below demonstrate that HSCs are ATP-secreting cells and that these cells serve as a target of an ATP secretion inhibitor.

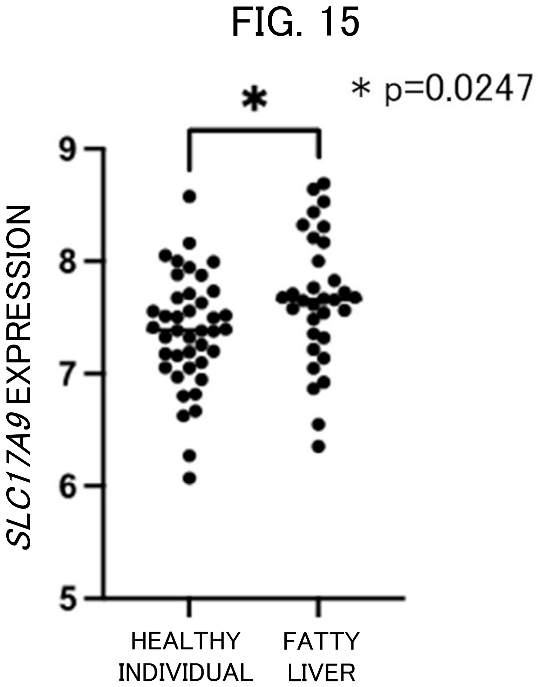

First, using microarray data from liver biopsies in obese patients (data set from the GEO database; GDS4881), expression of the *VNUT* gene (SLC17A9) was compared among groups. As healthy individuals, "Healthy Control" and "Healthy Obese" were included. As fatty liver patients, "NAFLD_steatohepatitis" and "NAFLD _steatosis" were included. As illustrated in FIG. 15, expression of SLC17A9 in the liver of fatty liver patients was statistically significantly increased relative to healthy individuals.

In order to demonstrate that VNUT is expressed in HSCs and that HSCs are ATP-secreting cells, the following experiment was carried out. Mouse liver samples fixed with 4% paraformaldehyde were embedded in paraffin, sectioned, and subjected to immunostaining using an α-smooth muscle actin (α-SMA) monoclonal antibody (1:500, catalog No. A5228, manufactured by Sigma-Aldrich) and a VNUT polyclonal antibody (1:250, catalog No. 26731-1-AP, manufactured by Proteintech).

Littermate wild-type mice (C57BL/6J) and VNUT knockout mice (Reference G: Shohei Sakamoto et al., 2014, Impairment of vesicular ATP release affects glucose metabolism and increases insulin sensitivity. Sci Rep., 4, 6689) were used. The mice were housed in a specific-pathogen-free facility (temperature: 22 to 24°C, humidity: 50 to 60%, 12-hour light/dark cycle) and fed either a standard solid diet (5.4% fat, CRF-1, manufactured by Oriental Yeast Co., Ltd.) or a high-fat diet (HFD) (24% fat, lard fat, 45 kcal% fat, D12451, manufactured by Research Diets, Inc.) ad libitum for 48 weeks. After 48 weeks, the mice were euthanized under ad libitum feeding conditions, and liver samples were collected. The liver samples were fixed in 4% paraformaldehyde, embedded in paraffin, and then sectioned.

Cells of the immortalized human hepatic stellate cell line LX-2 (manufactured by Merck) was cultured in Dulbecco's modified Eagle's medium (DMEM, 4500 mg/l Glucose, D5796, manufactured by Sigma-Aldrich) supplemented with inactivated 2% fetal bovine serum (FBS), 10 IU/ml penicillin, and 10 IU/ml streptomycin in a T25 or T75 flask at 37°C in 5% CO₂.

For immunofluorescence microscopy of LX-2 cells, cells on a coverslip were fixed with 4% paraformaldehyde in phosphate-buffered saline (PBS) at room temperature for 30 minutes. The cells were washed with PBS, and incubated for 20 minutes in the same buffer supplemented with 0.1% Triton X-100. Primary antibody treatment was performed using the following antibodies:
α-SMA monoclonal antibody (1:500, catalog No. A5228, manufactured by Sigma-Aldrich)
VNUT polyclonal antibody (1:250, catalog No. 26731-1-AP, manufactured by Proteintech)
vesicle-associated membrane protein (VAMP) 3 monoclonal antibody (1:200, catalog No. sc-514843, manufactured by Santa Cruz Biotechnology)
VAMP7 monoclonal antibody (1:200, catalog No. sc-166394, manufactured by Santa Cruz Biotechnology)
V-ATPase monoclonal antibody (1:200, catalog No. sc-55544, manufactured by Santa Cruz Biotechnology)
LAMP-1 monoclonal antibody (1:200, catalog No. SMC-140, manufactured by StressMarq Biosciences)

α-SMA is a fibrous protein with contractile ability that is expressed in the process of differentiation of fibroblasts into highly contractile myofibroblasts during the wound healing process. In the liver, α-SMA is present in HSCs, and its expression increases upon the activation. Thus, it serves as an indicator of activation and deactivation. VAMP has eight isoforms, 1 to 8, and forms part of SNARE, a membrane fusion apparatus. VMAP3 and VAMP7 are required for fusion and exocytosis of secretory granules and endosomes. LAMP-1 is an indicator protein for lysosomes.

Cell specimens were observed using a microscope (BZ-X700 series, manufactured by Keyence) or a confocal laser microscope (FV1000, manufactured by Olympus Corporation). For colocalization analysis, Pearson's correlation coefficients were calculated using the Coloc-2 analysis function of ImageJ.

In CLEM, wild-type mice were fed randomly and anesthetized with isoflurane. The liver was perfused in situ through the heart with 30 mM HEPES buffer supplemented with 100 mM NaCl and 2 mM CaCl₂, and then fixed at room temperature for 5 minutes in 4% formaldehyde prepared in the same buffer. Immediately thereafter, the tissue was cut into small blocks and further immersed for 4 hours. The sample was dehydrated using an ethanol gradient and embedded in LR White resin (manufactured by Polysciences). Subsequently, the embedded sample was cut into sections with a thickness of 1 µm using an ultramicrotome (Artos 3D, manufactured by Leica Microsystems), and then mounted on a glass slide. The sections were incubated with a VNUT polyclonal antibody (1:100, catalog No. 26731-1-AP, manufactured by Proteintech) at 4°C overnight, and then labeled with an Alexa 488-conjugated secondary antibody. The labeled sections were visualized using a confocal microscope (FV1000, manufactured by Olympus Corporation). Subsequently, the sections were stained with heavy metals (1% uranyl acetate and Sato's lead solution) and coated with a 2-nm layer of osmium (Neoc-Pro, manufactured by MeiwaFosis Co., Ltd.). Thereafter, the sections were observed using a scanning electron microscope (JSM-IT800, manufactured by JEOL Ltd.), and the area preliminarily observed by the confocal microscope was visualized.

### (Results)

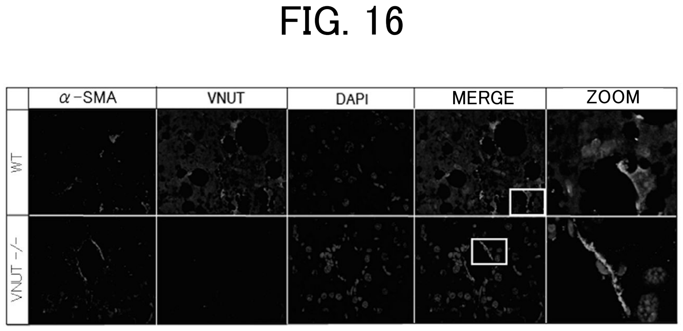

As illustrated in FIG. 16, the immunostaining revealed that positive sites for the VNUT antibody and the α-SMA monoclonal antibody coincided in the liver of the wild-type mice (WT). On the other hand, in the liver of the VNUT knockout mice (VNUT -/-), reactivity of the VNUT polyclonal antibody was absent. These findings indicated that VNUT is present prominently in HSCs.

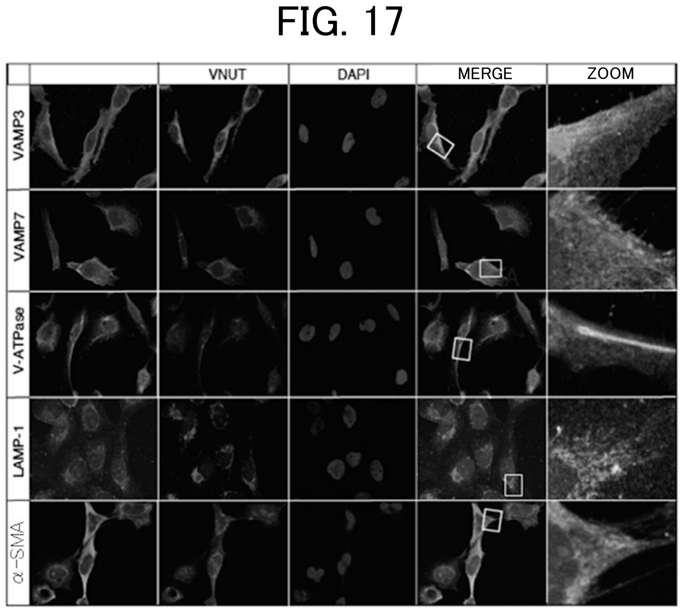

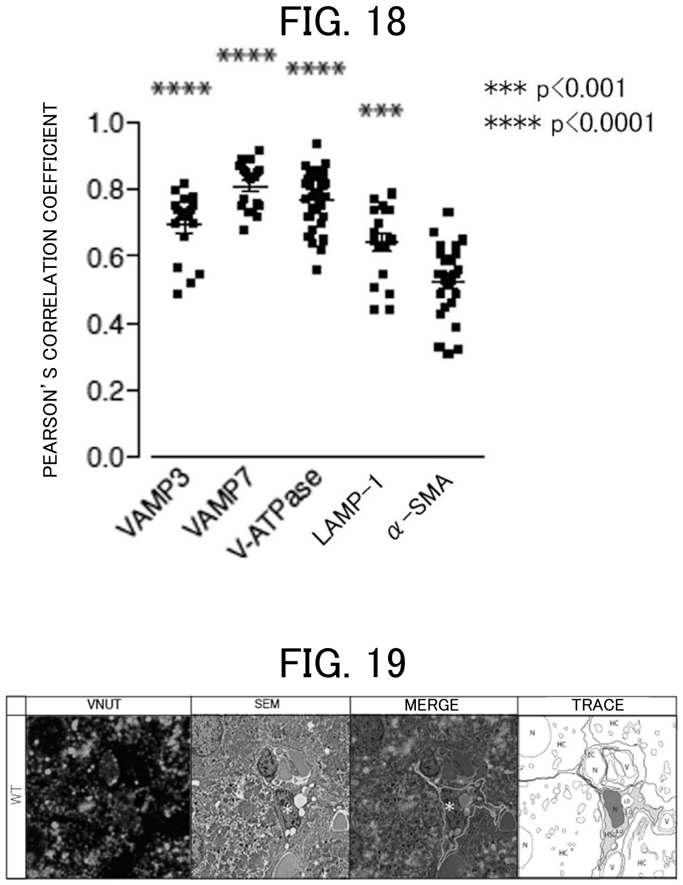

FIG. 17 illustrates images obtained by immunofluorescence microscopy of LX-2 cells. In the LX-2 cells, specific reaction of the VNUT polyclonal antibody showed a granular distribution in the cytoplasm, particularly around the nucleus. This reaction coincided well with the reaction sites of the VAMP3 monoclonal antibody and the V-ATPase monoclonal antibody. The Pearson's correlation coefficients between the localization of VNUT and the reactivity with α-SMA, VAMP3, VAMP7, V-ATPase, or LAMP-1 as obtained from FIG. 17 are illustrated in FIG. 18. The correlation coefficients were as high as about 0.8. These results suggest that VNUT is present in acidic secretory vesicles that contain V-ATPase.

FIG. 19 illustrates images obtained by CLEM. Expression of VNUT in HSCs could be confirmed at the electron microscopy level. Each asterisk in FIG. 19 indicates the nucleus of a stellate cell. VNUT was present in vesicles around intracellular lipid droplets.

### (Example 14 Study of Ca²⁺-dependent ATP secretion from LX-2 cells and the effect of an ATP secretion inhibitor)

LX-2 cells (2 × 10⁶ cells per well) were seeded in a collagen-coated 6-well culture dish containing Dulbecco's modified Eagle's medium (manufactured by Sigma-Aldrich) supplemented with 10 IU/ml penicillin, 10 IU/ml, and 2% fetal bovine serum, and cultured in 5% CO₂ at 37°C. After 3 days, the LX-2 cells were washed twice and preincubated in Krebs-Ringer buffer (10 mM HEPES-Tris, pH 7.4, 128 mM NaCl, 1.9 mM KCl, 1.2 mM KH₂PO₄, 1.3 mM MgSO₄, 26 mM NaHCO₃, 2.4 mM CaCl₂, 10 mM glucose, 0.2% (w/v) bovine serum albumin) with or without 10 µM clodronate. The LX-2 cells were then stimulated with Krebs-Ringer buffer supplemented with ionomycin (manufactured by Cayman) regardless of whether clodronate was present or not. For the stimulation, 1 mM EGTA (manufactured by Dojindo) or 10 µM serotonin (5-HT, manufactured by Sigma-Aldrich) was used. The ATP concentration in the culture supernatant was measured in the same manner as in Example 6.

For an siRNA experiment, a TriFECTa (trademark) RNAi Kit (manufactured by Integrated DNA Technologies) was used. A scramble RNA (scRNA) as a control siRNA, or a VNUT-specific siRNA, was incubated with Lipofectamine 3000 reagent (manufactured by Invitrogen) in OptiMEM reduced-serum medium (#31985070, manufactured by Gibco) at room temperature for 15 minutes, and then added directly to the cultured cells. The LX-2 cells as the transfection mixture were plated, and then cultured for 48 hours. The base sequences of VNUT siRNA forward and VNUT siRNA reverse, used for the above kit, are shown in SEQ ID NOs: 1 and 2, respectively. A positive control for an endogenous gene (#51-01-08-02) and a negative control (#51-01-14-03) were used.

### (Results)

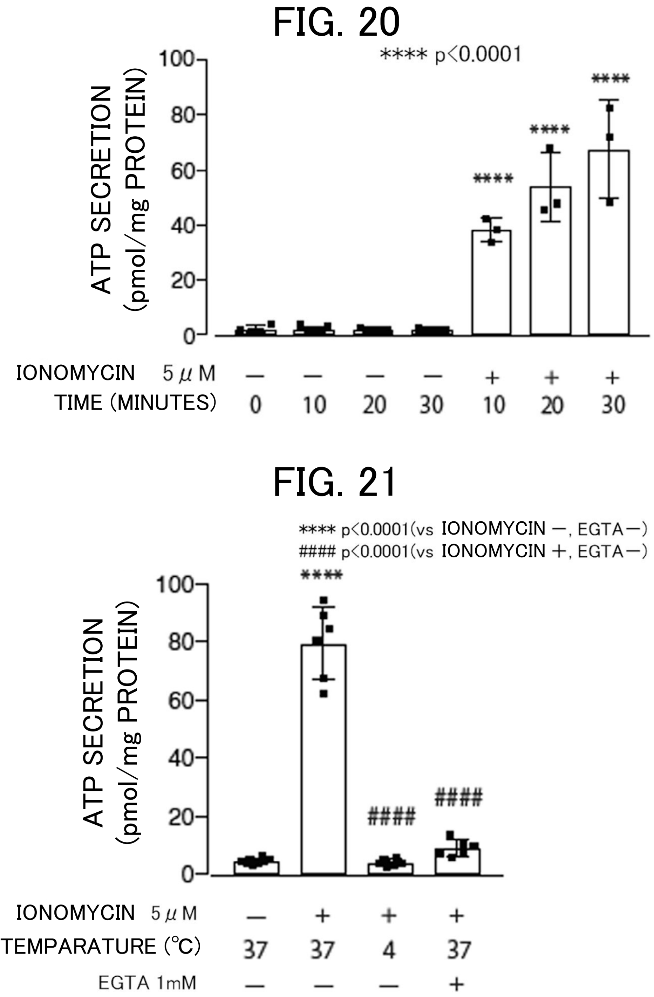

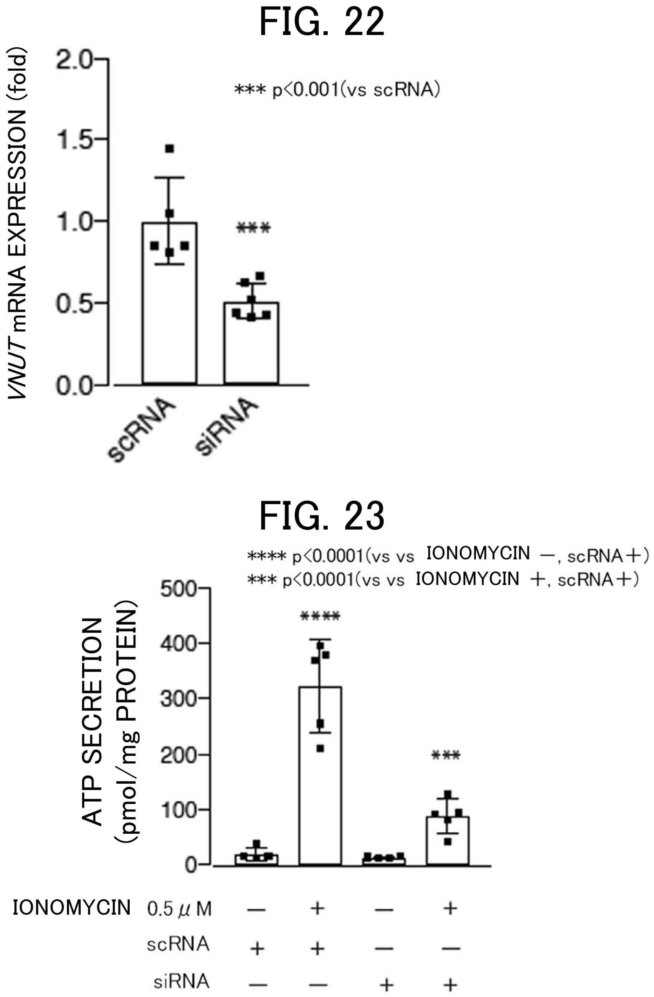

As illustrated in FIG. 20, addition of ionomycin resulted in increased secretion of ATP by the LX-2 cells over time (N = 3). As illustrated in FIG. 21, the ATP secretion required a certain temperature (37°C) and influx of Ca²⁺ into the cells. Chelating of Ca²⁺ with EGTA strongly inhibited the ATP secretion. No ATP secretion was found at 4°C, indicating that the secretion was temperature-dependent (N = 6). As illustrated in FIG. 22, the RNAi inhibited expression of the SLC17A9 gene in the LX-2 cells (N = 6). As illustrated in FIG. 23, inhibition of expression of the SLC17A9 gene inhibited the ATP secretion. On the other hand, the ATP secretion was not inhibited in the control experiment using the scramble RNA (N = 5). These results indicated that the ionomycin-induced ATP secretion in the LX-2 cells occurred by exocytosis via ATP-loaded vesicles.

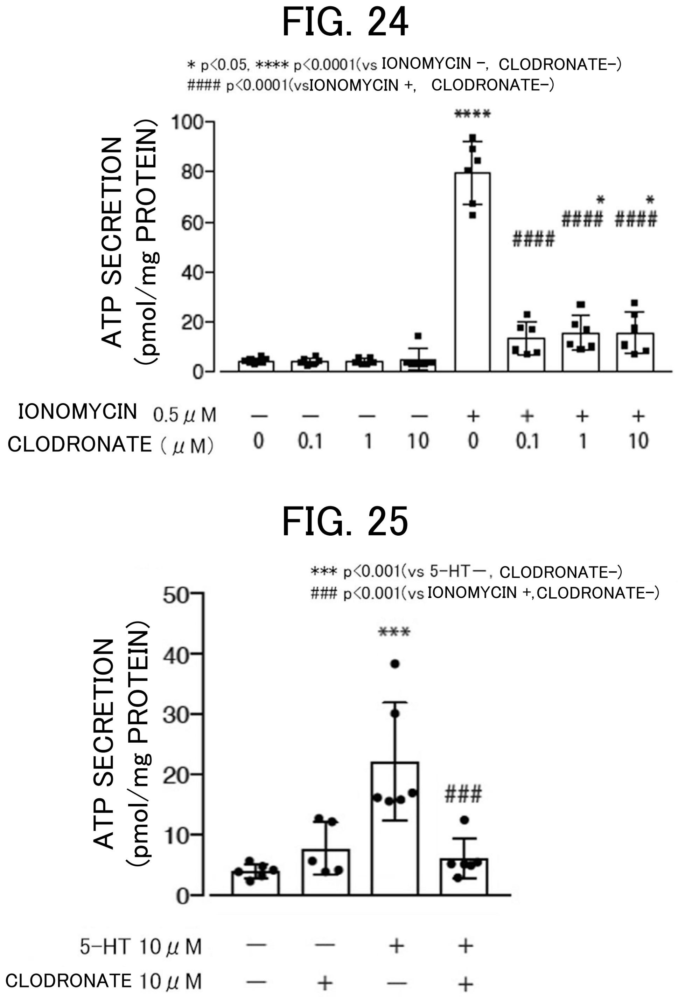

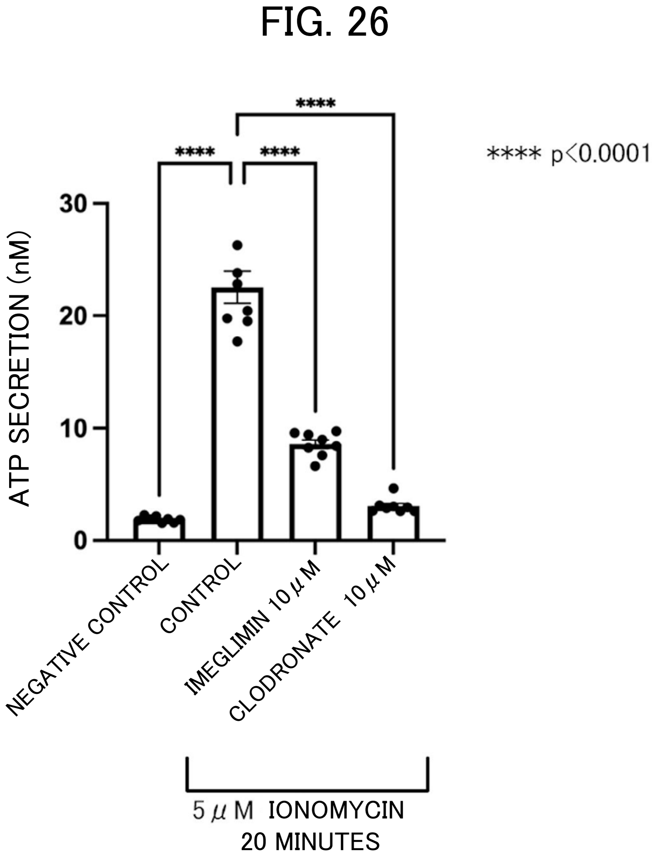

As illustrated in FIG. 24, ATP exocytosis was inhibited by clodronate, a VNUT inhibitor (N = 6). As illustrated in FIG. 25, 5-HT, which is a physiological substance that activates hepatic stellate cells, also promoted ATP secretion from the LX-2 cells. This secretion was strongly inhibited by clodronate (N = 6). As illustrated in FIG. 26, imeglimin inhibited ATP secretion from the LX-2 cells (N = 8).

### (Example 15 Thioacetamide-induced hepatic fibrosis and study of a therapeutic effect of imeglimin)

Wild-type mice, 2C57BL/6J (manufactured by Charles River Japan), were housed on a 12-hour light/dark cycle and fed a standard solid diet (5.4% fat, CRF-1, manufactured by Oriental Yeast Co., Ltd.) ad libitum. Thioacetamide (TAA, manufactured by FUJIFILM Wako Pure Chemical Corporation) was dissolved in physiological saline at 0.9% w/v. Eight-week-old mice were randomly assigned to three groups and continuously treated for 9 weeks according to the following scheme. At Week 9, all mice were euthanized 1 hour after administration of physiological saline or IMG.

Normal control group (N = 6): Physiological saline was intraperitoneally administered to mice three times per week. Eight hours after each administration, a vehicle was orally administered to the mice.

TAA group (N = 6): TAA(50 mg/kg, intraperitoneal) was administered to mice. Eight hours thereafter, physiological saline was orally administered to the mice. This treatment was performed three times per week for a total of 9 weeks.

TAA + IMG group (N = 6): During the first week, physiological saline was orally administered to mice. Thereafter, imeglimin (IMG) at 200 mg/kg was orally administered three times per week for 8 weeks. The administration of IMG was performed 8 hours after intraperitoneal administration of TAA (50 mg/kg).

For immunohistochemical analysis, liver samples were fixed in 4% paraformaldehyde, embedded in paraffin, and then sectioned. The sections were stained with HE or PSR. In addition, the paraffin-embedded sections were subjected to immunostaining using an α-SMA monoclonal antibody (1:500, catalog No. A5228, manufactured by Sigma-Aldrich) and an F4/80 monoclonal antibody (1:200, catalog No. MCA497GA, manufactured by AbD Serotec). The cell specimens were observed using a microscope (BZ-X700 series, manufactured by Keyence) or a confocal laser microscope (FV1000, manufactured by Olympus Corporation). The number of F4/80-positive cells was counted in four high-power fields per section. The α-SMA-positive area and the PSR-positive area were quantified using the image analysis software Fiji-ImageJ (1.54f).

A histological score was determined by a blinded investigator using the NAFLD histological scoring system of the Nonalcoholic Steatohepatitis Clinical Research Network. The total NAFLD activity score (NAS) was calculated from the total of the individual scores for steatosis (PSR staining), lobular inflammation (F4/80), and hepatocyte ballooning. The range of each parameter was 0 to 2 (ballooning) or 3 (the other two scores), where 0 indicates normal and 2 or 3 indicates severe. Staging of fibrosis was based on the NASH fibrosis stages, and a fibrosis stage ranging from 0 to 4 was determined according to Reference H (David E. Kleiner et al., 2005, Design and validation of a histological scoring system for nonalcoholic fatty liver disease, Hepatology, 41, 1313-1321).

All results are expressed as the mean ± standard error of the mean (S.E.M.). Statistical analyses were performed using GraphPad Prism 7.0 (GraphPad Software). Significance upon comparison between two groups was evaluated by an unpaired two-tailed Student's t-test. An unpaired two-tailed Student's t-test was used. Statistical significance among three or more groups was determined by two-way analysis of variance (ANOVA) with Tukey's or Dunnett's post hoc test. Coloc2 was used to perform image analysis, automatic thresholding, and pixel intensity correlation for statistical processing. Statistical significance was assumed when the P value was < 0.05.

### (Results)

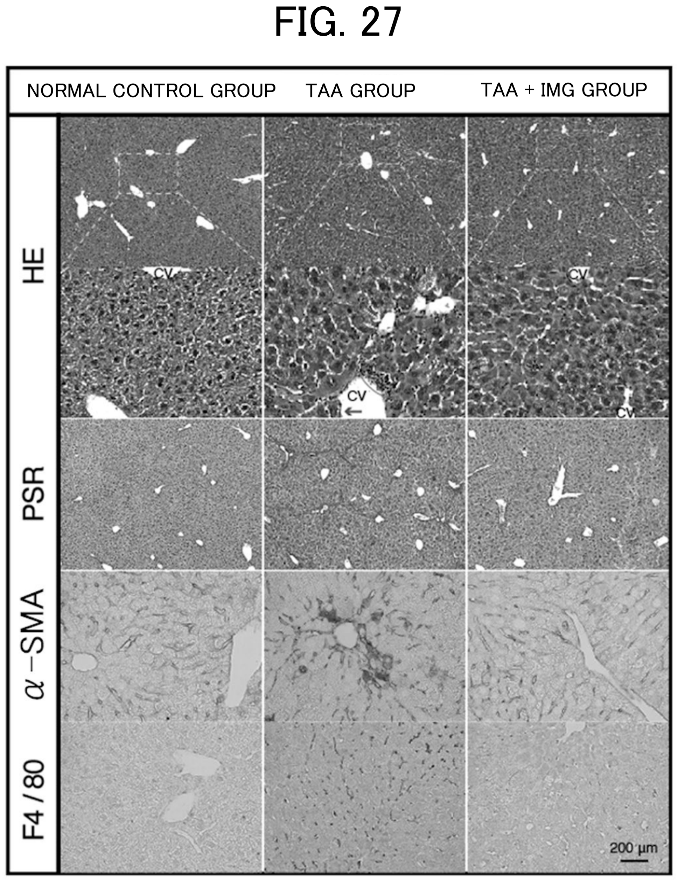

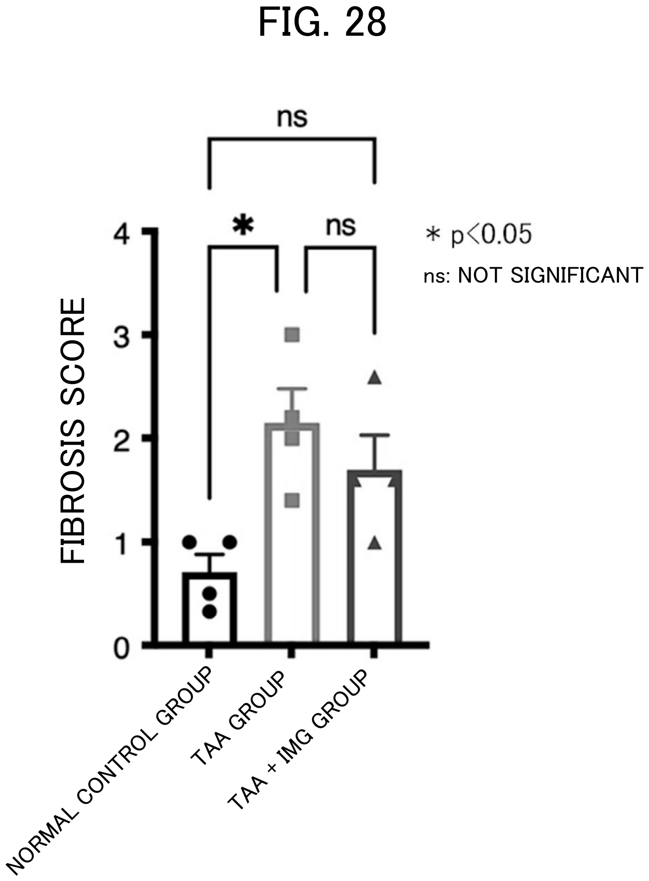

As illustrated in FIG. 27, the HE-stained liver tissue of the TAA group exhibited destruction of the lobular structure, hepatocyte ballooning, and inflammatory cell infiltration compared to that of the normal control group. The PSR staining-positive area, indicative of collagen fibers, was significantly reduced in the TAA + IMG group compared to the TAA group. The α-SMA-positive reaction, indicative of activation of hepatic stellate cells, was markedly increased by TAA particularly around the central and portal veins, and this increase was strongly suppressed by IMG. Positive reaction of F4/80-positive macrophages was also markedly increased by TAA, and this increase was strongly suppressed by IMG. As illustrated in FIG. 28, the fibrosis score was markedly increased in the TAA group relative to the normal control group. This increase was suppressed by IMG (TAA + IMG group). Administration of IMG reduced destruction of the lobular structure, hepatocyte ballooning, and inflammatory cell infiltration, thereby reducing the fibrosis stage.

### (Example 16 TAA-induced activation of HSCs, and inhibition of HSC activation and induction of HSC deactivation by an ATP secretion inhibitor)

In accordance with Example 15, immunostaining of α-SMA in liver sections and observation by fluorescence microscopy were performed for the normal control group, the TAA group, and the TAA + IMG group, and for a TAA + Clo group, which was treated in the same manner as the TAA + IMG group except that clodronate, instead of imeglimin, was administered at 30 mg/kg by blending it in the drinking water from 1 week after the start of TAA administration.

Further, α-SMA was compared by Western blotting. Fresh mouse liver was homogenized in Western lysis buffer (20 mM Tris-HCl, pH 7.6, 150 mM NaCl, 2 mM EDTA, 0.5% NP-40) supplemented with protease and phosphatase inhibitor tablets (manufactured by Roche Applied Science), followed by centrifugation at 10,000×g at 4°C for 5 minutes to obtain a supernatant. Protein content in the supernatant was quantified using a BCA Protein Assay Kit (manufactured by ThermoFisher Scientific). The sample was mixed with Laemmli sample buffer (1:1, manufactured by Bio-Rad) and heated at 95°C for 5 minutes, followed by applying 40 µg of the heated mixture to e-PAGEL E-R520L (manufactured by ATTO) and performing electrophoresis (SDS-PAGE) in the presence of sodium dodecyl sulfate. Proteins in the gel were transblotted onto a PVDF membrane, reacted with primary antibodies, and then reacted with a horseradish peroxidase-conjugated secondary antibody. As primary antibodies, an α-SMA monoclonal antibody (1:500, catalog number A5228, manufactured by Sigma-Aldrich) and a β-actin monoclonal antibody (catalog number 4970-s, manufactured by Cell Signaling) were used. Thereafter, protein bands were visualized using an electrochemiluminescence (ECL) Western blotting detection system (manufactured by GE Healthcare) and detected with an image analyzer LAS-4000 mini (manufactured by Fujifilm).

### (Results)

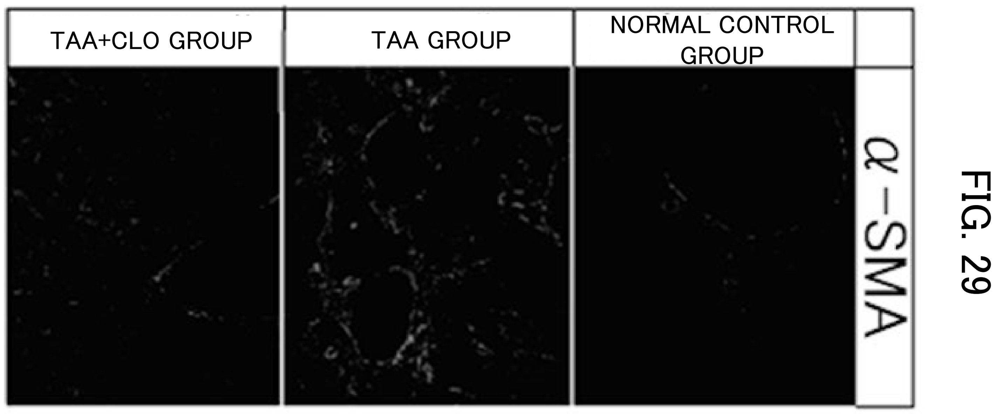

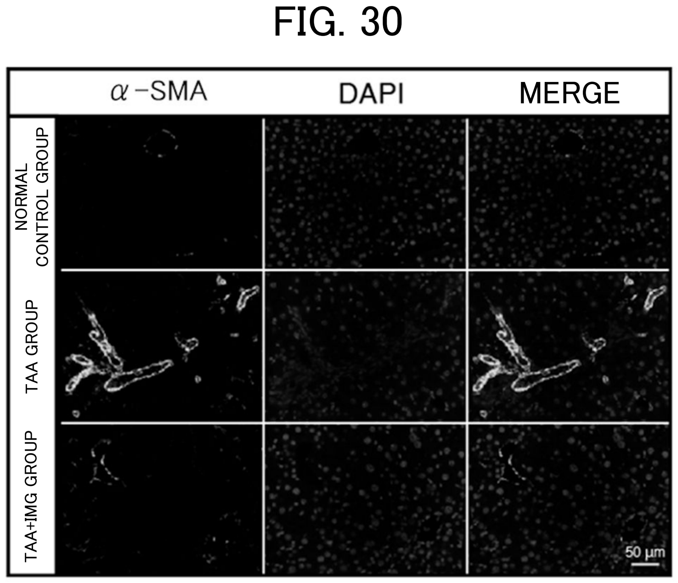

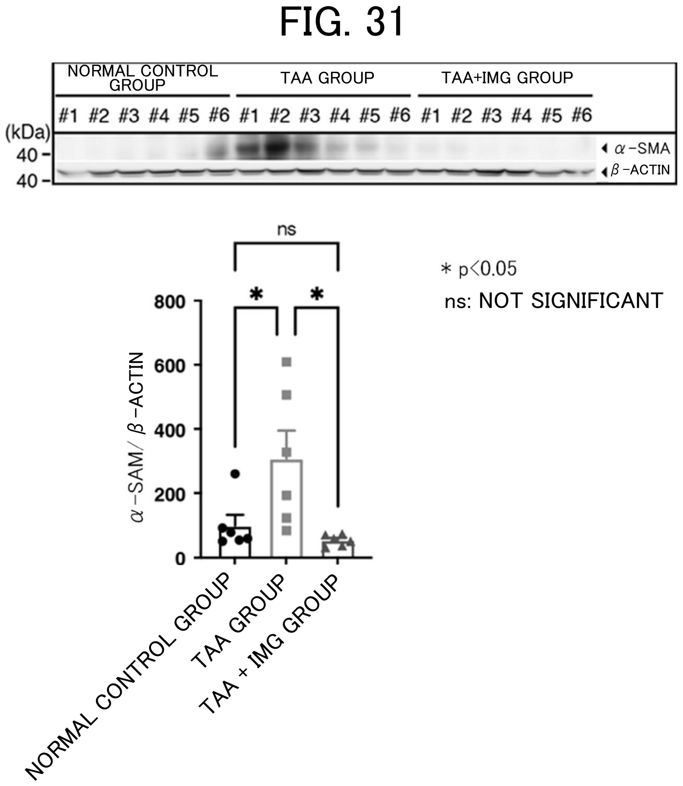

α-SMA-positive cells (stellate cells) are mainly located around the central and portal veins in mouse liver tissue. As illustrated in FIG. 29, α-SMA-positive reaction increased markedly throughout the mouse liver in the TAA group, but this increase was markedly suppressed by clodronate. The α-SMA-positive area fraction was 0.14 ± 0.01 in the normal control group, 2.79 ± 0.33 in the TAA group, and 0.71 ± 0.10 in the TAA + Clo group (four times of measurement per group). As illustrated in FIG. 30, imeglimin exhibited an effect similar to that of clodronate. FIG. 31 illustrates the result of Western blotting of the liver tissue. The Western blotting also confirmed that the increase in α-SMA-positive reaction due to TAA was markedly suppressed by imeglimin. These results indicated that an ATP secretion inhibitor inhibits HSC activation and induces HSC deactivation.

### (Example 17 Study of imiquimod-induced enhancement of ATP secretion in keratinocytes and its inhibition by imeglimin)

In the same manner as in Example 1, keratinocytes were cultured at 2.5×10⁴ cells/well in a 24-well plate at 37°C in 5% CO₂ for 4 days. The medium was replaced with HBSS (manufactured by Gibco) supplemented with 1 µM or 10 µM imeglimin, and incubation was performed at 37°C in 5% CO₂ for 18 hours. Thereafter, imiquimod was added thereto at 1 µg/ml or 0.5 µg/ml, and incubation was performed for 15 minutes, followed by collection of 200 µl of the supernatant. The supernatant was immediately treated on a heat block at 95°C for 10 minutes, and ATP in the supernatant was quantified using a ATP Assay Kit - Luminescence (manufactured by Dojindo) (N = 8).

### (Results)

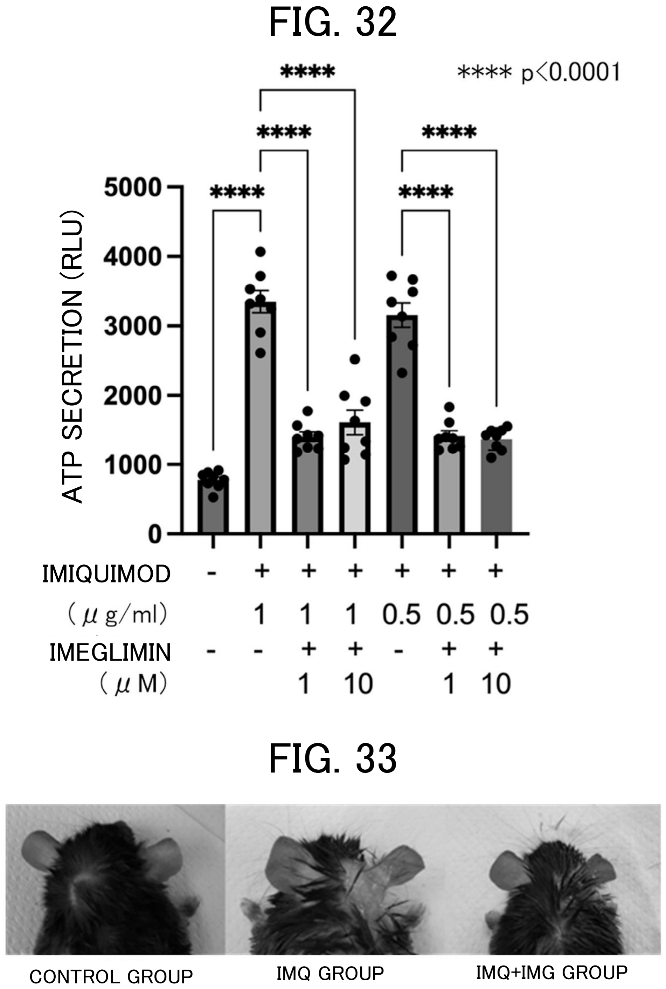

As illustrated in FIG. 32, ATP secretion from the keratinocytes was markedly promoted by imiquimod. This ATP secretion was strongly inhibited by 1 µM and 10 µM imeglimin.

### (Example 18 Study of the action of imeglimin in an imiquimod-induced psoriasis model)

Experimental psoriatic symptoms were induced in a mouse auricle by imiquimod. Six-week-old male C57BL/6 mice (N = 5) were acclimated for one week. On Days 1 to 7, 62.5 mg of 5% imiquimod cream (Beselna Cream 5%) (3.125 mg in terms of the active ingredient, manufactured by Mochida Pharmaceutical Co. Ltd.) was applied to the right auricle of the mice for 7 consecutive days (IMQ group). Once daily after the imiquimod application, the imeglimin administration group (IMQ + IMG group) received oral administration, at 40 mg/kg, of 500-mg Twymeeg (trademark) tablets (manufactured by Sumitomo Pharma Co. Ltd.) dissolved in physiological saline. The control group received an equal volume of physiological saline. On Day 8, the mice were euthanized by cervical dislocation, and auricle thickness was measured using a dial thickness gauge (manufactured by Adachi Sogyo Co., Ltd.).

Each excised mouse auricle was fixed in 4% paraformaldehyde at 4°C overnight. After ethanol substitution, the auricle was embedded in paraffin, sectioned using a rotary microtome Leica RM2165 (thickness, 5 mm), and subjected to HE staining or immunohistochemical staining. For the immunohistochemical staining, the sections were deparaffinized, washed in xylene, and rehydrated through graded alcohols. The sections were incubated in DAKO at 95°C for 30 minutes. The sections were blocked at room temperature for 60 minutes and then incubated at room temperature for 2 hours to allow reaction with a TGM1 anti-rabbit polyclonal antibody (catalog number 12912-3-AP, manufactured by Proteintech). The sample was washed and then allowed to react with a secondary antibody (Alexa 544-conjugated secondary antibody (goat anti-rabbit IgG) (manufactured by Molecular Probes)) at room temperature for 60 minutes. After allowing reaction with DAPI at room temperature for 10 minutes, the sections were mounted with Fluoromount and imaged using a fluorescence microscope (BZ-X710, manufactured by Keyence).

### (Results)

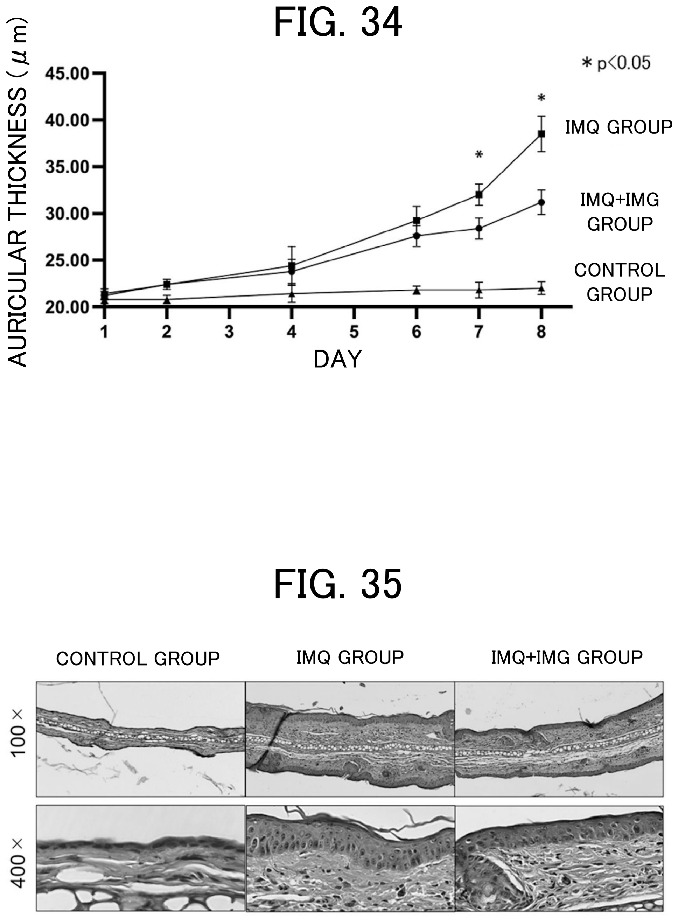

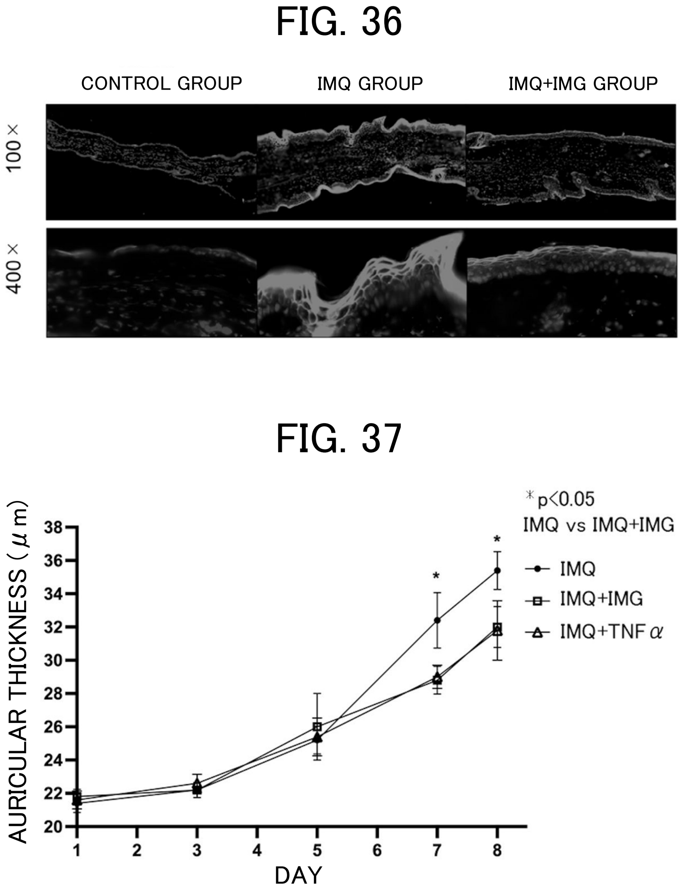

As illustrated in FIG. 33, direct application of imiquimod to the auricle caused psoriatic symptoms in the epidermis, leading to partial desquamation. Oral administration of imeglimin eliminated epidermal desquamation and reduced the psoriatic symptoms. As illustrated in FIG. 34, auricle thickening induced by the direct application of imiquimod to the auricle was suppressed by oral administration of imeglimin. FIG. 35 and FIG. 36 illustrate HE-stained tissues and the localization of TMG1-positive cells, respectively. TMG1 is an enzyme expressed during a series of processes in which keratinocytes (epidermal keratinocytes) proliferate in the basal layer, progress through the spinous layer and the granular layer to the stratum corneum, and finally shed as scales. This enzyme constitute the epidermal sheet structure. TMG1 serves as an indicator of the skin barrier function. The inhibitory effect of imeglimin on psoriatic symptoms was also supported by the immunohistochemical images of TMG1, an epidermal marker.

The present Example demonstrated that imeglimin suppresses the development of psoriasis.

### (Example 19 Action of imeglimin in an imiquimod-induced psoriasis model and comparison with an anti-TNFα antibody)

The dorsal hair of mice was shaved and depilated (Day 0). To each of the left auricle and the depilated back of each mouse, 62.5 mg of 5% imiquimod cream (Beselna Cream 5%) was applied once daily a total of seven times (Days 1 to 7) in accordance with Example 18, to induce imiquimod-induced psoriasis (IMQ group). In the IMQ + IMG group, mice received oral administration of imeglimin (40 mg/kg) once daily (Day 1 to 7) after the imiquimod application. In the IMQ + TNFα group, 100 µg/mouse (corresponding to 5 mg/kg in patients with psoriasis) of infliximab (manufactured by Nichi-Iko Pharmaceutical Co., Ltd.), an anti-TNFα antibody as an existing drug, was intravenously injected on Days 1, 3, and 5 (N = 5). Thickness of the auricle was measured, and the degrees of erythema, desquamation, and infiltration in the affected area were rated on Days 1, 3, 5, 7, and 8 according to the PASI scoring of the DermNET Dermatology Resource (none: 0, mild: 1, moderate: 2, severe: 3, and very severe: 4). The resulting values were used to calculate the PASI score.

Further, for an auricle excised on Day 8, immunohistochemical staining was performed in the same manner as in Example 18, and auricular sections were imaged using a fluorescence microscope (BZ-X710; manufactured by Keyence Corporation).

### (Results)

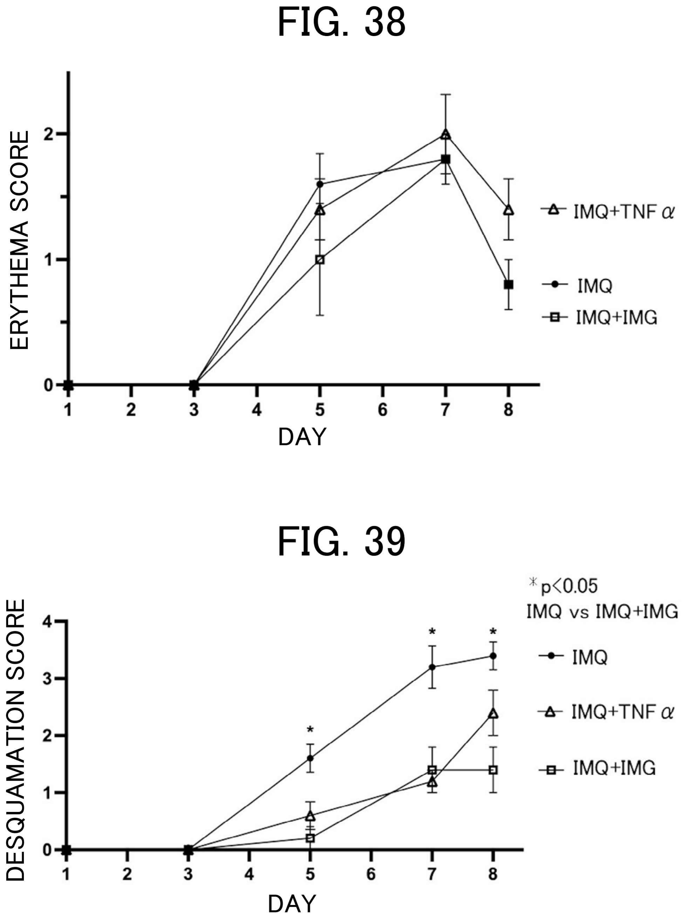

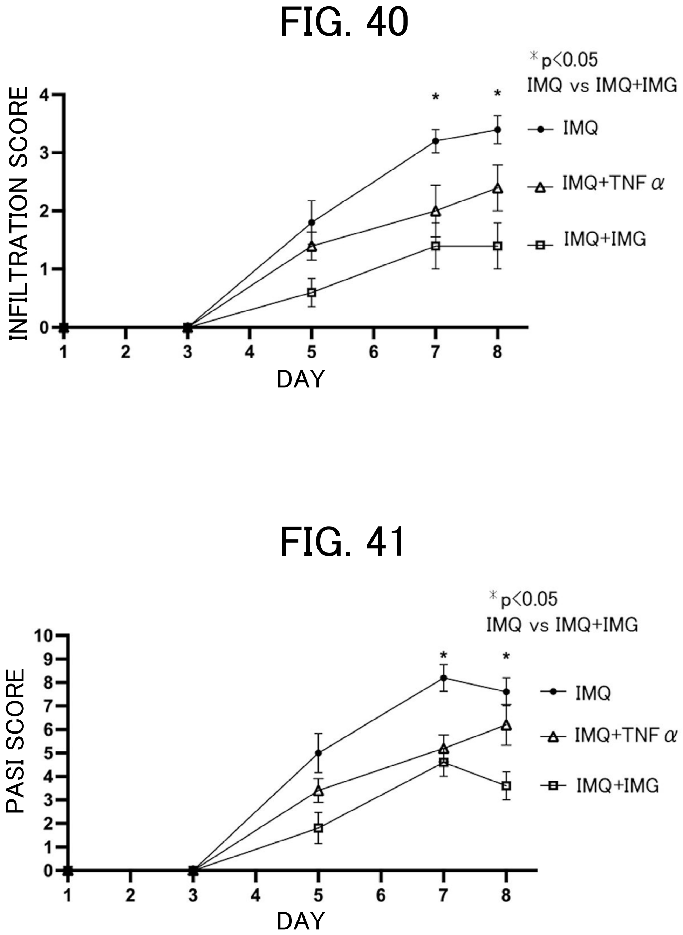

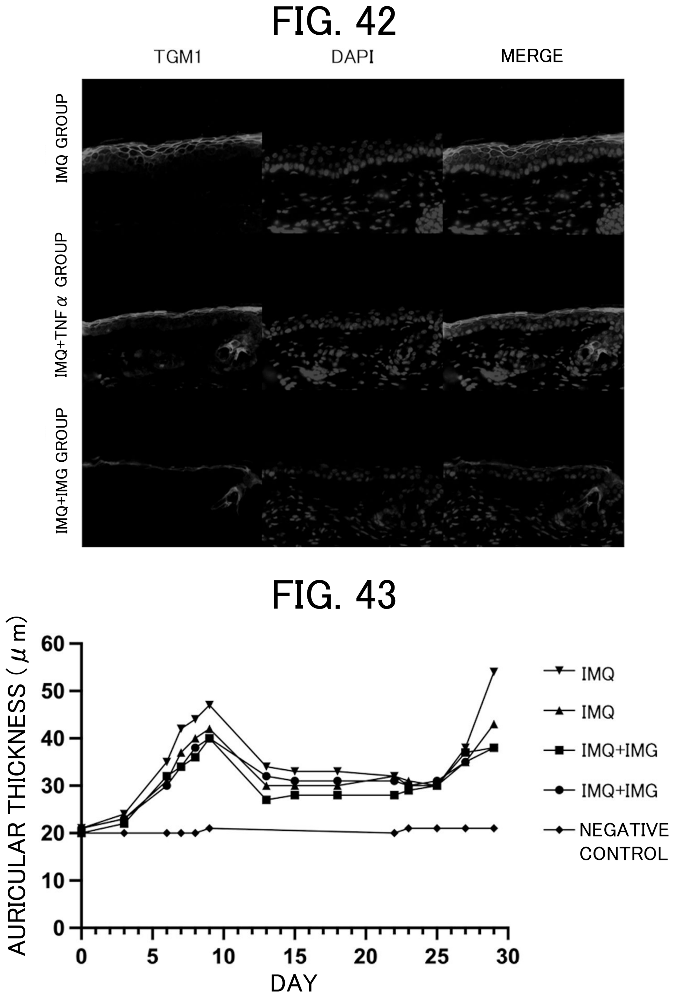

FIGS. 37, 38, 39, 40, and 41 illustrate time-course changes in the auricular thickness, the erythema score, the desquamation score, the infiltration score, and the PASI score, respectively. Typical psoriatic symptoms induced by imiquimod treatment, except erythema, were reduced by infliximab and imeglimin. FIG. 42 illustrates immunohistochemical images (×400) of the respective auricular sections. The inhibitory effects of infliximab and imeglimin on psoriatic symptoms were also supported by the immunohistochemical images of TMG1, an epidermal marker. The effects of infliximab and imeglimin were almost equivalent, or imeglimin was more effective.

### (Example 20 Study of relapse-preventing effect of imeglimin in an imiquimod-induced psoriasis relapse model)

To the left auricle of each mouse, 62.5 mg of 5% imiquimod cream (Beselna Cream 5%) was applied once daily a total of seven times (Days 1 to 7) in accordance with Example 19 to induce imiquimod-induced psoriasis. Thereafter, the imiquimod treatment was interrupted, and imeglimin (40 mg/kg) was orally administered on alternate days for 2 weeks (Day 8 to 22) (IMG). Mice without administration of imeglimin were designated IMQ. On Day 23, the imiquimod treatment was resumed, and 62.5 mg of 5% imiquimod cream (Beselna Cream 5%) was applied once daily a total of seven times (Days 23 to 27). In parallel, imeglimin (40 mg/kg) was orally administered daily to IMG.

### (Results)

FIG. 43 illustrates changes in the auricular thickness over time. The interruption of the imiquimod treatment led to a rapid decrease in psoriatic symptoms, and, by Day 11, symptoms had almost disappeared to reach remission. Subsequently, when the imiquimod treatment was resumed on Day 23, psoriatic symptoms were induced more rapidly and more strongly than at the first induction. On the other hand, in the mice in which the imiquimod treatment was resumed while imeglimin was concomitantly administered, the symptoms were markedly reduced compared to IMQ. The present Example demonstrated that imeglimin has a relapse-preventing effect on psoriasis.

### (Example 21 Study of the effects of topical clodronate and imeglimin solutions on imiquimod-induced psoriasis)

On Days 1, 2, 3, 5, and 6, 62.5 mg of 5% imiquimod cream (Beselna Cream 5%) was applied to auricles of wild-type mice (IMQ) or VNUT knockout mice (VNUT KO + IMQ) to induce psoriatic symptoms. On Day 5 and later, the auricles exhibited psoriatic symptoms and became thickened (N = 2). Application of 20% glycerin solution (glycerin lotion) after the imiquimod application similarly induced psoriatic symptoms (IMQ + Gly). For a negative control, only 20% glycerin solution was applied. In IMQ + 1% IMG and IMQ + 0.1% IMG, 20% glycerin solution supplemented with 1% (w/v) imeglimin and 0.1% (w/v) imeglimin, respectively, was applied immediately after the imiquimod application. Further, in IMQ + 1% Clo and IMQ + 0.1% Clo, 20% glycerin solution supplemented with 1% (w/v) clodronate and 0.1% (w/v) clodronate, respectively, was applied immediately after the imiquimod application.

### (Results)

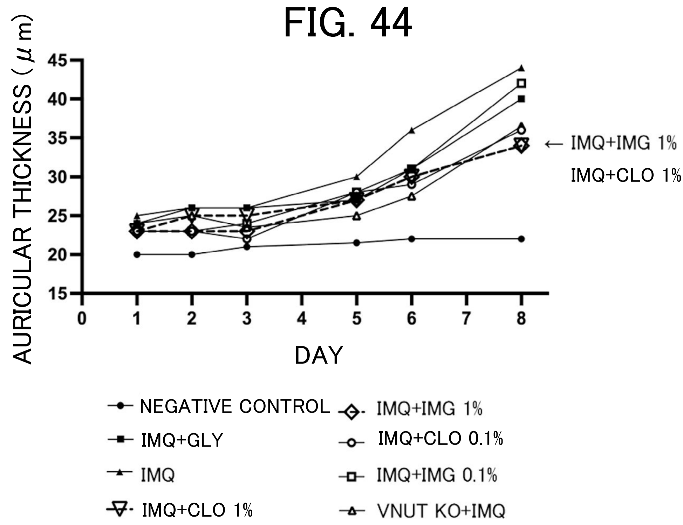

FIG. 44 illustrates changes in the auricular thickness over time. No auricular thickening was found in the negative control. In IMQ + 1% IMG, IMQ + 1% Clo, and IMQ + 0.1% Clo, the degree of auricular thickening was improved, and the psoriatic symptoms were reduced. The effect of imeglimin was concentration-dependent, and no effect was found with IMQ + 0.1% IMG. Induction of psoriasis by imiquimod was weaker in the VNUT knockout mice, and the auricular thickness of these mice was about the same as in IMQ + 1% Clo, IMQ + 0.1% Clo, and IMQ + 1% IMG. These results indicated that ATP secretion is required for the psoriasis-inducing effect of imiquimod and that topical application of an ATP secretion inhibitor is effective in psoriasis treatment.

This application claims the benefit of Japanese Patent Application No. 2023-074235, filed on April 28, 2023, the entire disclosure of which is incorporated by reference herein.

### Industrial Applicability

The present disclosure is useful for inhibition of ATP secretion in purinergic chemical transmission.

## Claims

1. An adenosine triphosphate secretion inhibitor comprising:
imeglimin, metformin, phosphoenolpyruvic acid, an analog of phosphoenolpyruvic acid, or a pharmaceutically acceptable salt thereof.

2. The adenosine triphosphate secretion inhibitor according to claim 1,
for use in prevention, amelioration, or treatment of a disease or symptom related to purinergic chemical transmission.

3. The adenosine triphosphate secretion inhibitor according to claim 1,
wherein the analog is L-2-phosphoglyceric acid, D-2-phosphoglyceric acid, or 3-phosphoglyceric acid.

4. A hepatic stellate cell activation inhibitor comprising:
imeglimin, metformin, phosphoenolpyruvic acid, an analog of phosphoenolpyruvic acid, or a pharmaceutically acceptable salt thereof.

5. A hepatic stellate cell deactivator comprising:
imeglimin, metformin, phosphoenolpyruvic acid, an analog of phosphoenolpyruvic acid, or a pharmaceutically acceptable salt thereof.

6. A hepatic fibrosis therapeutic agent comprising:
imeglimin, metformin, phosphoenolpyruvic acid, an analog of phosphoenolpyruvic acid, or a pharmaceutically acceptable salt thereof.

7. A non-alcoholic steatohepatitis therapeutic agent comprising:
imeglimin, metformin, phosphoenolpyruvic acid, an analog of phosphoenolpyruvic acid, or a pharmaceutically acceptable salt thereof.

8. A psoriasis therapeutic agent comprising:
imeglimin, metformin, phosphoenolpyruvic acid, an analog of phosphoenolpyruvic acid, or a pharmaceutically acceptable salt thereof.

9. Use of imeglimin, metformin, phosphoenolpyruvic acid, an analog of phosphoenolpyruvic acid, or a pharmaceutically acceptable salt thereof, for production of an adenosine triphosphate secretion inhibitor, a hepatic stellate cell activation inhibitor, a hepatic stellate cell deactivator, a hepatic fibrosis therapeutic agent, a non-alcoholic steatohepatitis therapeutic agent, or a psoriasis therapeutic agent.
